# FASCICULE DE BREVET EUROPEEN

(11) **EP 1 692 281 B1**
(45) Date de publication et mention de la délivrance du brevet: **26.12.2007**
(21) Numéro de dépôt: 05815749.6
(22) Date de dépôt: 26.10.2005
(51) Int. Cl.: C12N 9/12, G01N 33/53, C12Q 1/68

(54) **IDENTIFICATION D'UNE MUTATION DE JAK2 IMPLIQUEE DANS LA POLYGLOBULIE DE VAQUEZ**
IDENTIFIZIERUNG EINER, IN DER POLYCYTHEMIA VERA IMPLIZIERTEN, JAK2 MUTANTE
IDENTIFICATION OF A JAK2 MUTATION IN POLYCYTHEMIA VERA

(30) Priorité: 27.10.2004 FR 0411480
(43) Date de publication de la demande: 23.08.2006
(60) Demande divisionnaire: 07118874.2
(73) Titulaire: Assistance Publique - Hopitaux de Paris, 75004 Paris (FR); INSTITUT NATIONAL DE LA SANTE ET DE LA RECHERCHE MEDICALE (INSERM), 75013 Paris (FR); Institut Gustave Roussy, 94800 Villejuif (FR); Université de Versailles Saint-Quentin-en-Yvelines, 78035 Versailles Cedex (FR); Université Paris Sud, 91400 Orsay (FR)
(72) Inventeur: VAINCHENKER, William, 75005 Paris (FR); UGO, Valérie, 75011 Paris (FR); JAMES, Chloé, 92120 Montrouge (FR); LE COUEDIC, Jean-Pierre, 77420 Champs sur Marne (FR); CASADEVALL, Nicole, 75013 Paris (FR)
(74) Mandataire: Warcoin, Jacques
(86) Numéro de dépôt international: PCT/EP2005/055586
(87) Numéro de publication internationale: WO 2006/045827

(56) Documents cités:
- US-A- 5 914 393
- DATABASE UniProt [Online] 7 juin 2005 (2005-06-07), "Janus kinase 2." XP002369149 extrait de EBI accession no. UNIPROT:Q506Q0 Database accession no. Q506Q0
- JAMES CHLOE ET AL: "A unique clonal JAK2 mutation leading to constitutive signalling causes polycythaemia vera" NATURE (LONDON), vol. 434, no. 7037, avril 2005 (2005-04), pages 1144-1148, XP002369147 ISSN: 0028-0836
- THE CANCER GENOME PROJECT BAXTER E J ET AL: "Acquired mutation of the tyrosine kinase JAK2 in human myeloproliferative disorders" LANCET THE, LANCET LIMITED. LONDON, GB, vol. 365, no. 9464, 19 mars 2005 (2005-03-19), pages 1054-1061, XP004798459 ISSN: 0140-6736
- UGO V ET AL: "Multiple signaling pathways are involved in erythropoietin-independent differentiation of erythroid progenitors in polycythemia vera" EXPERIMENTAL HEMATOLOGY, NEW YORK, NY, US, vol. 32, no. 2, février 2004 (2004-02), pages 179-187, XP002324352 ISSN: 0301-472X
- SALTZMAN ALAN ET AL: "Cloning and characterization of human Jak-2 kinase: High mRNA expression in immune cells and muscle tissue" BIOCHEMICAL AND BIOPHYSICAL RESEARCH COMMUNICATIONS, vol. 246, no. 3, 29 mai 1998 (1998-05-29), pages 627-633, XP002324351 ISSN: 0006-291X
- PAHL H L: "TOWARDS A MOLECULAR UNDERSTANDING OF POLYCYTHEMIA RUBRA VERA" EUROPEAN JOURNAL OF BIOCHEMISTRY, BERLIN, DE, vol. 267, no. 12, juin 2000 (2000-06), pages 3395-3401, XP000982035 ISSN: 0014-2956

## Description

La présente invention concerne le variant V617F de la protéine tyrosine kinase JAK2, ledit variant étant responsable de la Polyglobulie de Vaquez. L'invention se rapporte également à une méthode de diagnostic en première intention de l'érythrocytose et de la thrombocytose permettant de les rattacher à des syndromes myéloprolifératifs ou à la détection dans les syndromes myéloprolifératifs du variant JAK2 V617F permettant de les reclasser dans un nouveau groupe nosologique et à l'identification d'inhibiteurs spécifiques et de siRNA.

La polyglobulie de Vaquez (Polycythemia Vera ou PV) est un syndrome myéloprolifératif chronique, associant une polyglobulie vraie et, souvent, une thrombocytose et une hyperleucocytose. Il s'agit d'une maladie acquise, clonale, de la cellule souche hématopoïétique. Les progéniteurs hématopoïétiques de PV sont capables de former des colonies érythroblastiques en l'absence d'érythropoïétine (Epo), appelées « colonies spontanées ». Une hypersensibilité des progéniteurs érythroblastiques de PV à plusieurs autres facteurs de croissance a également été démontrée : Interleukine-3 (IL-3), Granulocyte Macrophage-Stimulating Factor (GM-CSF), Stem Cell Factor (SCF), et Insuline Like Growth Factor (IGF-1). Plusieurs équipes se sont intéressées à la physiopathologie de la PV, mais l'anomalie moléculaire à l'origine de la maladie est restée inconnue à ce jour (H. Pahl, 2000).

L'hypersensibilité des progéniteurs de PV à plusieurs cytokines conduit à rechercher des anomalies touchant les voies de transduction du signal communes aux récepteurs aux cytokines. L'existence d'un marqueur moléculaire n'a jamais été mise en évidence dans la PV, mais étant données les similitudes entre la PV et les autres syndromes myéloprolifératifs, en particulier la LMC, il paraît probable que des mécanismes moléculaires proches de ceux induits par Bcr-Ab1 soient responsables de l'avantage de prolifération du clone malin et de sa différenciation terminale. Cette hypothèse a été confirmée récemment dans deux syndromes myéloprolifératifs rares, les syndromes myéloprolifératifs associés à une translocation impliquant la région chromosomique 8p11 qui induit une activation constitutive du récepteur du FGF et le syndrome hyperéosinophilique, dans lequel une délétion chromosomique cryptique aboutit à un gène chimérique PDGFRα-FIP1L1. Dans les deux cas, les anomalies moléculaires sont à l'origine de protéines de fusion ayant une activité tyrosine kinase constitutive.

Dans la PV, il n'a pas été trouvé d'anomalie cytogénétique récurrente, même si une délétion 20q est détectée chez 10 à 15% des patients, et une perte d'hétérozygotie en 9p dans environ 30% des cas (Kralovics, 2002). Cependant, ces anomalies ne sont pas spécifiques de la maladie.

Les cellules de PV présentant une indépendance à l'Epo, des recherches ont été entreprises sur la voie du récepteur de l'Epo (R-Epo). Tout d'abord, le récepteur est normal tant sur le plan structurel que fonctionnel (Hess et al., 1994; Le Couedic et al.; 1996; Means et al., 1989). La phosphatase SHP-1 qui déphosphoryle R-Epo et JAK2 à l'arrêt de la stimulation par l'Epo, est normalement exprimée au niveau ARN et protéique (Andersson et al., 1997; Asimakopoulos et al., 1997). Plus en aval dans la signalisation de R-Epo, une activation anormale de STAT5 a été recherchée dans les polynucléaires (PNN) de patients présentant une PV sans retrouver d'anomalie. En revanche, une phosphorylation constitutive de STAT3 a été mise en évidence dans les PNN de 4 cas de PV sur 14 étudiés (Roder, 2001). Enfin, l'expression de la protéine anti-apoptotique bcl-xl, cible transcriptionnelle de STAT5, a été étudiée en immunohistochimie et en cytométrie de flux (Silva et al., 1998). Il a ainsi été montré que bcl-xl était hyperexprimée dans les érythroblastes de PV et notamment à un stade plus mature où cette protéine n'est normalement plus exprimée.

Dans la polyglobulie de Vaquez, les principaux critères diagnostiques sont à ce jour cliniques (critères du PVSG; Pearson, 2001). Le diagnostic biologique repose essentiellement sur la réalisation des cultures de progéniteurs érythroïdes en l'absence d'Epo (détection des colonies endogènes). En raison de l'expertise nécessaire à sa bonne réalisation, et l'importance du « temps-technicien » dépensé, cet examen n'est pas disponible dans tous les centres, et n'est fiable que lorsqu'il est réalisé par un laboratoire expérimenté. De plus, le test nécessite d'être réalisé sur cellules médullaires prélevées sur le patient pour une bonne sensibilité, ce qui est n'est pas anodin pour le patient.

Par des techniques d'hybridation soustractive, une équipe allemande a cloné un gène hyperexprimé dans les PNN de PV, appelé PRV1 (Polycythemia Rubra Vera 1) (Temerinac et al., 2000). La protéine PRV-1 appartient à la superfamille des récepteurs de surface uPAR. L'hyperexpression de l'ARNm codant pour PRV-1 dans les polynucléaires de PV est facilement détectable par RT-PCR en temps réel, et constitue un marqueur de la maladie de découverte récente, sans rôle physiopathologique. Cependant, les études récemment publiées montrent qu'il n'est ni très sensible ni très spécifique.

Spivak JL et al., en 2003 (« Chronic myeloproliferative disorders. » ; Hematology; 2003;200 24.) décrit certains marqueurs de la PV. Les mRNA de l'antigène neutrophile NBI/CD177 sont surexprimés dans les granulocytes de patients PV. Ce marqueur ne semble toutefois pas un moyen fiable de détecter les PV, certains malades ne présentant pas cette surexpression ou cette surexpression pouvant être observée sur des sujets atteints de syndromes myéloprolifératifs autres que la Polyglobulie de Vaquez. Une expression diminuée du récepteur à la thrombopoïetine, Mpl, sur les plaquettes est également retrouvée dans les PV. Bien que cette anomalie prédomine dans la PV, elle est retrouvée dans d'autres syndromes myéloprolifératifs. En outre, il s'agit d'un examen difficile à réaliser qui ne peut être effectué que dans des laboratoires spécialisés.
Ainsi, il n'existe dans l'état de la technique aucune méthode permettant un diagnostic fiable de la PV. En outre, les seuls traitements disponibles ne sont pas spécifiques. Il s'agit de saignées pour maintenir l'hématocrite dans les limites de la normale, ou l'emploi d'agents cytotoxiques, ou encore d'IFN.

Dans le cadre de la présente invention, nous avons non seulement découvert une mutation dans le gène JAK2 dans environ 90% des patients testés mais nous avons également établi que cette mutation est responsable d'une activation constitutive de cette tyrosine kinase et démontré que son inhibition permet de bloquer la prolifération et la différenciation spontanées des érythroblastes de PV.

La séquence de JAK2 est décrite dans la Database Uniprot sous le numéro Q506Q0.

Des mutations dans Jak2 ont été décrites un an après la présente invention dans James Chloe et al, Nature, vol. 434, n° 7037, p. 1144 et Baxter et al, lancet, vol. 365, et n° 9464 p. 1054.

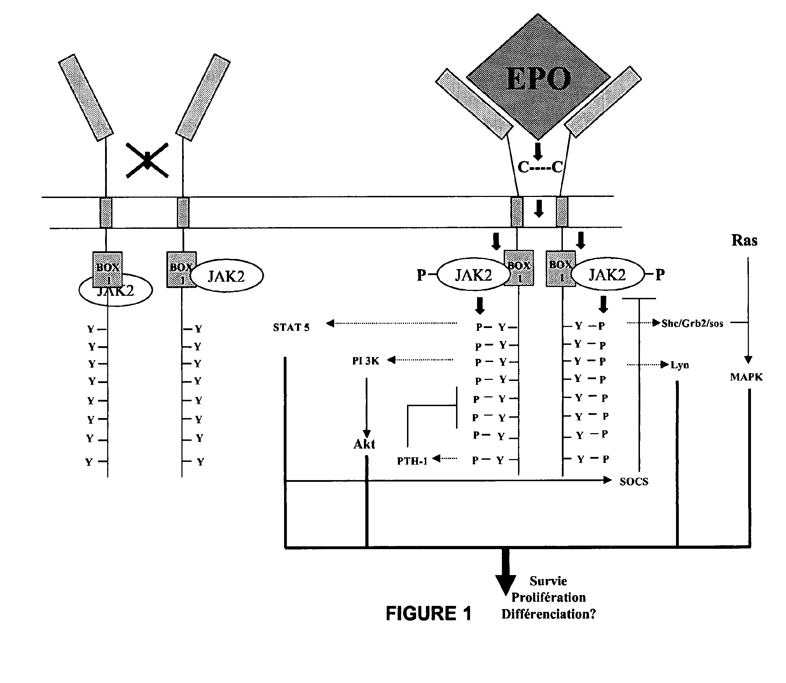

JAK2 appartient à la famille des Janus Kinases (JAKs), qui regroupe plusieurs tyrosine kinases intracytoplasmiques : JAK1, JAK2, JAK3 et TYK2. Les protéines JAK sont impliquées dans la signalisation intracellulaire de nombreux récepteurs membranaires qui n'ont pas d'activité tyrosine kinase intrinsèque, comme certains membres de la superfamille des récepteurs aux cytokines et en particulier le récepteur à l'Epo (R-Epo). La protéine JAK2 est codée par un gène qui comporte 23 exons. L'ADN complémentaire a une taille de 3500 paires de bases, et code pour une protéine de 1132 acides aminés (130 kD) (Figure 1). Nous avons identifié par PCR et séquençage une mutation ponctuelle acquise et clonale dans l'exon 12 de JAK2 chez près de 90 % des patients atteints de PV. Le codon 617 « GTC », codant normalement une Valine (V), est muté en « TTC », codant une Phénylalanine (F). Cette mutation V617F n'est pas retrouvée chez les 25 témoins ou patients atteints de polyglobulie secondaire testés. Par contre elle est retrouvée dans 40% des thrombocytémies essentielles et dans 50% des myélofibroses si bien que cette mutation définit un nouveau cadre de syndrome myéloprolifératif comme Bcr-Abl a défini la leucémie myéloïde chronique.

Pour étudier si le variant de l'invention JAK2 V617F pourrait être efficacement détecté avec des instruments largement répandus classiquement utilisés dans les laboratoires de diagnostic en hématologie, nous avons analysés 119 échantillons provenant de patients avec une suspicion de maladie myéloproliférative. Nous avons montré que JAK2 V617F était efficacement détectée par les technologies LightCycler® et TaqMan®, ces dernières étant un peu plus sensibles que le séquençage. Nous avons ensuite estimé la valeur de détection de JAK2 V617F en tant que test de diagnostic en première intention chez 88 patients avec des taux d'hématocrites supérieurs à 51%, et montré que la mutation correspondait au diagnostic de PV selon les critères WHO (R = 0,879) et PVSG (R = 0,717), avec une valeur prédictive positive de 100% dans le contexte de l'érythrocytose. Sur la base de ces données, nous proposons que la détection de JAK2 V617F dans les granulocytes soit considérée comme un test de diagnostic en première intention chez des patients avec une érythrocytose, évitant par conséquent la réalisation d'une mesure de la masse de globules rouges, d'un aspirat de moelle osseuse et d'une analyse *in vitro* de la formation de colonies érythroïdes endogènes. Cette détection pourra aussi être étendue en première intention à l'ensemble des syndromes myéloprolifératifs ou à leur suspicion. Cette détection sera particulièrement importante dans les thrombocytoses chroniques pour lesquelles il n'existe pas de tests biologiques certains pour affirmer un syndrome myéloprolifératif. Elle sera également un examen important dans le diagnostic des myélofibroses et devant des tableaux cliniques associées à des thromboses d'étiologie indéterminée.

Ainsi, l'invention apporte pour la première fois un outil de diagnostic et ouvre la voie au traitement ciblé de la PV et également des syndromes myéloprolifératifs associées à cette mutation. Plus spécifiquement, nous proposons la détection de la mutation JAK2 V617F en tant que test de diagnostic en première intention dans le cadre de l'érythrocytose, ce qui permet d'éviter la quantification de la masse de globules rouges et des cellules endogènes érythroïdes (EEC) d'un aspirat de la moelle osseuse dans la majorité des patients et dans les thrombocytoses chroniques ce qui permettra d'éviter une longue enquête étiologique.

### Description de l'invention

Ainsi, dans un premier aspect, la présente invention concerne la protéine isolée JAK2 (Janus kinase 2), en particulier la protéine Homo sapiens Janus kinase 2 (NCBI accession number NM_004972 ; GI:13325062), comprenant une mutation sur l'acide aminé 617 (codon 617 de l'ADNc à compter de l'ATG), plus particulièrement la mutation **V617F**, ci-après dénommée variant JAK2 V617F, telle que présentée à la SEQ ID No 1 ci-dessous :

### SEQ ID No 1 (V617F Homo sapiens Janus kinase 2 ou JAK2 V617F)

L'invention vise également des équivalents de cette protéine mutée en position 617 chez d'autres mammifères, par exemple les JAK2 V617F chez d'autres mammifères tels que le rat (NM_031514), le porc, la souris (NM_008413),... ainsi que des variants de la SEQ ID No 1 qui comportent en outre une ou plusieurs modifications qui n'affectent pas l'activité et la structure 3D du variant.

L'invention porte également sur une séquence nucléotidique codant pour la SEQ ID No 1, de préférence la SEQ ID No 2 (séquence du gène humain JAK2 avec le codon TTC au lieu de GTC sur le codon 617 (**mutation g/t en position 1849**, si après dénommée par G 1849T, à partir de l'ATG marquant le début de la traduction).

Cette séquence peut se trouver dans un vecteur viral ou plasmidique ou encore un ADN nu sous le contrôle d'un promoteur efficace dans les cellules de mammifères. L'invention s'étend donc à un vecteur exprimant la protéine JAK2 V617F.
Le vecteur de l'invention peut être un vecteur de clonage et/ou d'expression et peut être utilisé pour transfecter une cellule hôte, notamment une cellule de mammifère, à l'exception des cellules souches humaines embryonnaires ou des cellules germinals humaines de préférence une cellule progénitrice CD34⁺ humaine.

### Animal transgénique non-humain modèle de la PV et autres syndromes myéloprolifératifs

L'invention porte également sur un animal transgénique non humain exprimant JAK2 V617F recombinante. Cet animal peut de préférence être une souris ou un rat. Les rats ou souris transgéniques servant de modèle peuvent être obtenus par toute méthode couramment utilisée par l'homme du métier, notamment un Knock-in (insertion ciblée d'une séquence) par recombinaison homologue ou recombinaison dirigée avec les système Cre-LoxP ou FLP-FRT dans des cellules ES. Selon un mode préféré de réalisation de l'invention, la cellule transgénique selon l'invention est obtenue par ciblage génique (« gene targeting ») du variant JAK2 G1849T au niveau d'une ou des séquences du génome de la cellule hôte. Plus précisément, le transgène est inséré de manière stable par recombinaison homologue au niveau de séquences homologues dans le génome de la cellule hôte. Lorsqu'il s'agit d'obtenir une cellule transgénique en vue de produire un animal transgénique, la cellule hôte est de préférence une cellule souche embryonnaire non-humaine (cellule ES) (Thompson *et al.,* 1989). Le ciblage génique représente la modification dirigée d'un locus chromosomique par recombinaison homologue avec une séquence d'ADN exogène ayant une homologie de séquence avec la séquence endogène ciblée. On distingue différents types de ciblage génétique. Ici, le ciblage génique peut être utilisé plus particulièrement pour remplacer le gène JAK2 sauvage par le gène variant JAK2 G1849T ou toute autre variant génétiquement similaire. Dans ce cas, le ciblage génique est appelé « Knock-in » (K-in). Alternativement, le ciblage génique peut être utilisé pour diminuer ou annihiler l'expression de JAK2 sauvage puis pour introduire le gène du variant de JAK2. Il s'agit alors de ciblage génique appelé « Knock-Out » (KO) (voir Bolkey *et al.,* 1989). La cellule selon l'invention se caractérise en ce que le transgène est intégré de manière stable dans le génome de ladite cellule, et en ce que son expression est contrôlée par les éléments de régulation du gène endogène. Par intégration de manière stable, on entend signifier l'insertion du transgène dans l'ADN génomique de la cellule selon l'invention. Le transgène ainsi inséré est ensuite transmis à la descendance cellulaire. L'intégration du transgène est réalisée en amont, en aval ou au milieu du gène endogène cible JAK2. Facultativement, un ou plusieurs gènes de sélection positive ou négative peuvent être utilisés. On peut aussi utiliser des régions d'ADN d'homologie avec le locus cible, de préférence au nombre de deux, situé de part et d'autre de la portion du gène rapporteur ou de part et d'autres de la séquence complète à insérer. Par « régions d'ADN d'homologies », on entend désigner deux séquences d'ADN qui, après un alignement optimal et après comparaison, sont identiques pour habituellement au moins environ 90% à 95% des nucléotides et, préférentiellement, au moins environ 98 à 99,5% des nucléotides. L'alignement optimal des séquences pour la comparaison peut être réalisé au moyen de l'algorithme d'homologie locale de Smith et Waterman (1981), au moyen de l'algorithme d'homologie locale de Neddleman et Wunsch (1970), au moyen de la méthode de recherche de similarité de Pearson et Lipman (1988), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Bien que aussi peu que 14 pb homologues à 100% soient suffisantes pour réaliser la recombinaison homologue dans les bactéries et les cellules de mammifère, des portions de séquences homologues plus longues sont préférées (en général ces portions font au moins 2000 pb, de préférence au moins 5 000 pb pour chaque portion de séquence homologue. Avantageusement, la séquence JAK variant est insérée dans l'ensemble des éléments assurant une régulation du type endogène, c'est-à-dire un ensemble comprenant au moins le promoteur, des séquences régulatrices (enhanceurs, silenceurs, insulateurs), les signaux de terminaison du gène JAK endogène.

Selon un mode de réalisation particulier, le transgène JAK G1849T comprend au moins la séquence codante, une cassette de sélection positive encadrée ou non de sites-spécifiques à l'action des recombinases, par exemple une cassette Lox/Néo-TK/L.ox ou lox/Néo/lox ou FRT/Néo-TK/FRT ou FRT/Néo/FRT pouvant être également présente en position 5' de ladite séquence, et caractérisé en ce qu'une cassette de sélection négative contenant par exemple le ou les gènes DTA et/ou TK est présente à au moins une des extrémités du transgène. Le transgène de la présente invention est de préférence dérivé directement d'une séquence d'ADN exogène présente naturellement dans une cellule animale. Cette séquence d'ADN sous forme native peut être modifiée par exemple par insertion de sites de restriction nécessaires au clonage et/ou par insertion de sites de recombinaison site spécifiques (séquences lox et flp).

A cet effet, le variant JAK2 G 1849T peut être cloné dans un vecteur de clonage qui permet d'en assurer sa propagation dans une cellule hôte, et/ou facultativement dans un vecteur d'expression pour assurer l'expression du transgène. Les technologies de l'ADN recombinant utilisées pour la construction du vecteur de clonage et/ou d'expression selon l'invention sont connues de l'homme du métier. Les techniques standard sont utilisées pour le clonage, l'isolement de l'ADN, l'amplification, et la purification ; les réactions enzymatiques impliquant l'ADN ligase, l'ADN polymérase, les endonucléases de restriction sont effectuées selon les recommandations du fabricant. Ces techniques et les autres sont généralement réalisées selon Sambrook *et al.,* 1989). Les vecteurs incluent des plasmides, les cosmides, les bactériophages, les rétrovirus et autres virus animaux, les chromosomes artificiels, tels les YAC, BAC, HAC et autres vecteurs analogues.

Les méthodes pour générer des cellules transgéniques selon l'invention sont décrites dans Gordon *et al.,* 1989. Diverses techniques pour transfecter des cellules de mammifères ont été revues par Keon *et al.,* 1990. Le transgène selon l'invention, facultativement compris dans un vecteur linéarisé ou non, ou sous la forme d'un fragment de vecteur, peut être introduit dans la cellule hôte par des méthodes standard telles que par exemple la micro-injection dans le noyau (US 4,873,191), la transfection par précipitation au phosphate de calcium, la lipofection, l'électroporation (Lo, 1983), le choc thermique, la transformation avec des polymères cationiques (PEG, polybrène, DEAE-Dextran...), ou encore l'infection virale (Van der Putten *et al.,* 1985).

Lorsque les cellules ont été transformées par le transgène, elles peuvent être cultivées *in vitro* ou bien être utilisées pour produire des animaux transgéniques non humains. Après transformation, les cellules sont ensemencées sur une couche nourricière et/ou dans un milieu approprié. Les cellules contenant la construction peuvent être détectées en utilisant un milieu sélectif. Après un temps suffisant pour laisser les colonies pousser, celles-ci sont récupérées et analysées pour déterminer si un événement de recombinaison homologue et/ou une intégration de la construction se sont produits. Pour réaliser le criblage des clones susceptibles de satisfaire à la recombinaison homologue, des marqueurs positifs et négatifs, encore appelés gènes de sélection, peuvent être insérés dans le vecteur de recombinaison homologue. Différents systèmes de sélection des cellules ayant réalisé l'événement de recombinaison homologue ont été décrits (pour revue US 5 627 059). Ledit gène de sélection positive selon l'invention est de préférence choisi parmi les gènes de résistance aux antibiotiques. Parmi les antibiotiques, on peut citer de manière non exhaustive la néomycine, la tétracycline, l'ampicilline, la kanamycine, la phléomycine, la bléomycine, l'hygromycine, le chloramphénicol, la carbénicilline, la généticine, la puromycine. Les gènes de résistance correspondants à ces antibiotiques sont connus de l'homme du métier ; à titre d'exemple, le gène de résistance à la néomycine rend les cellules résistantes à la présence de l'antibiotique G418 dans le milieu de culture. Le gène de sélection positif peut également être sélectionné parmi le gène HisD, l'agent sélectif correspondant étant l'histidinol. Le gène de sélection positif peut également être sélectionné parmi le gène de la guanine-phosphoribosyl-transférase (GpT), l'agent sélectif correspondant étant la xanthine. Le gène de sélection positif peut également être sélectionné parmi le gène de l'hypoxanthine-phosphoribosyl-transférase (HPRT), l'agent sélectif correspondant étant l'hypoxanthine. Le ou les marqueurs de sélection utilisés pour permettre d'identifier les événements de recombinaison homologue peuvent affecter par la suite l'expression génique, et peuvent être éliminés, si nécessaire, par la mise en oeuvre de recombinases site spécifiques telle la recombinase Cre spécifique des sites Lox (Sauer, 1994 ; Rajewsky *et al.,* 1996; Sauer, 1998) ou FLP spécifique des sites FRT (Kilby *et al.,* 1993).

Les colonies positives, c'est-à-dire contenant des cellules dans lesquelles au moins un événement de recombinaison homologue s'est produit sont identifiées par une analyse par southern blotting et/ou par des techniques de PCR. Le taux d'expression, dans les cellules isolées ou les cellules de l'animal transgénique selon l'invention, de l'ARNm correspondant au transgène peut également être déterminé par des techniques comprenant l'analyse par northern blotting, l'analyse par hybridation *in situ,* par RT-PCR. Egalement les cellules ou tissus animaux exprimant le transgène peuvent être identifiés en utilisant un anticorps dirigé contre la protéine rapporteuse. Les cellules positives peuvent ensuite être utilisées pour réaliser les manipulations sur l'embryon et notamment l'injection des cellules modifiées par recombinaison homologue dans les blastocystes.

Pour ce qui concerne la souris, les blastocystes sont obtenus à partir de femelles superovulées de 4 à 6 semaines. Les cellules sont trypsinées et les cellules modifiées sont injectées dans le blastocèle d'un blastocyste. Après l'injection, les blastocystes sont introduits dans la corne utérine de femelles pseudo-gestantes. On laisse ensuite les femelles aller jusqu'à leur terme et les portées résultantes sont analysées pour déterminer la présence de cellules mutantes possédant la construction. L'analyse d'un phénotype différent entre les cellules de l'embryon nouveau-né et les cellules du blastocyste ou des cellules ES permet de détecter les nouveau-nés chimériques. Les embryons chimériques sont ensuite élevés jusqu'à l'âge adulte. Les chimères ou animaux chimériques, non-humains sont des animaux dans lesquels seule une sous-population de cellules possède un génome altéré. Les animaux chimériques présentant le gène ou les gènes modifiés, sont en général croisés entre eux ou avec un animal non-humain de type sauvage afin d'obtenir une descendance hétérozygote ou homozygote. Les hétérozygotes mâles et femelles sont ensuite croisés pour générer des animaux homozygotes. A moins qu'il ne soit indiqué, l'animal transgénique non humain selon l'invention comprend des changements stables de la séquence nucléotidique des cellules de la lignée germinale.

Selon un autre mode de réalisation de l'invention, la cellule transgénique non humaine selon l'invention peut servir de cellule donneuse de noyau dans le cadre d'un transfert de noyau ou transfert nucléaire. Par transfert nucléaire, on entend désigner le transfert de noyau d'une cellule vivante donneuse de vertébré, d'un organisme adulte ou au stade foetal, dans le cytoplasme d'une cellule receveuse énucléée de la même espèce ou d'une espèce différente non-humaine. Le noyau transféré est reprogrammé pour diriger le développement des embryons clonés qui peuvent ensuite être transférés dans des femelles porteuses pour produire les foetus et les nouveau-nés, ou utilisés pour produire des cellules de la masse cellulaire interne en culture. Différentes techniques de clonage nucléaire sont susceptibles d'être utilisées ; parmi celles-ci, il convient de citer de manière non exhaustive celles qui font l'objet des demandes de brevet WO 95 17500, WO 97/07668, WO 97 07669, WO 98 30683, WO 99 01163 et WO 99 37143.

Ainsi, l'invention s'étend également à un animal transgénique non humain comprenant une séquence recombinante codant pour JAK2 V617F. Ces animaux peuvent être homozygotes ou hétérozygotes (JAK2 V617F / JAK2 V617F ou JAK2 V617F / JAK2). Ces animaux reproduisent notamment la polyglobulie de Vaquez, mais également tout syndrome myéloprolifératif induit par JAK2 V617F. Ils permettent donc de réaliser un criblage fonctionnel d'inhibiteurs de tyrosine kinase, notamment un criblage d'inhibiteurs spécifiques de JAK2 V617F.
Une autre alternative peut consister à injecter un vecteur viral (rétrovirus ou lentivirus ou autres) capable d'exprimer le variant JAK2 V617F dans des cellules souches hématopoïétiques, non-humaines des cellules progénitrices non humaines ou des cellules ES non humaines pour également réaliser des modèles de polyglobulie de Vaquez ou d'autres syndromes myéloprolifératifs.

### Outils de diagnostic

Dans un troisième aspect, l'invention vise les outils de diagnostic permettant de détecter la présence ou l'absence de la mutation JAK2 V617F chez un mammifère atteint ou susceptible de présenter un syndrome myéloprolifératif, notamment chez les patients montrant une polyglobulie ou dont on suspecte qu'ils présentent les symptômes de la polyglobulie de Vaquez, des thrombocytémies et/ou des myélofibroses.

A ce titre, l'invention se rapporte aux amorces et sondes permettant de détecter la présence ou l'absence de la mutation dans la séquence SEQ ID No 2 décrite ci-dessus.

Plus particulièrement, l'invention a trait à un acide nucléique isolé ayant une séquence d'au moins 10, 12, 15, 20, 30, 40 ou encore 50 nucléotides consécutifs (par exemple de 10 à 30 nucléotides ou de 10 à 25 nucléotides) de l'exon 12 ou de la séquence SEQ ID No 3 ou 4 ci-dessous et comprenant le nucléotide muté t¹⁸⁴⁹ par exemple de 10 à 30 nucléotides.

### SEQ ID No 3

La séquence soulignée désigne un exemple de zones en amont ou en aval permettant le design de sondes ou d'amorces spécifiques de la mutation en position 1849 (SEQ ID No 4).

### Exemple de différentes amorces et sondes préférées de l'invention :

### SUR ADN, AMORCES DE PCR :

### SUR ADN, AMORCES DE SEQUENÇAGE :

### SUR CDNA, AMORCES DE PCR ET SEQUENÇAGE :

SENS 5'-CAACCTCAGTGGGACAAAGAA-3' (1386-1407) (SEQ ID No 9)
ANTI-SENS 5'-GCAGAATATTTTTGGCACATACA-3' (2019-2401) (SEQ ID No 10)

### SONDES SNPS ET DETECTION DE MUTATION ET SIRNA (1829-1870) :

TTTTAAATTATGGAGTATGTGTCTGTGGAGACGAGAATATTC (SEQ ID No 11)

### GENOTYPAGE sur LightCycler (ADN de PNN ou de moche)

Oligo "S"' (sens) GGCAGAGAGAATTTTCTGAAC (SEQ ID No 15)
Oligo "R" (antisens) GCTTTCCTTTTTCACAAGATA (SEQ ID No 16)
Sensor wt GTCTCCACAGACACATACTCCATAA 3'-FL (SEQ ID No 17)
Anchor JAK2 5'-LC Red640AAAACCAAATGCTTGTGAGAAAGCT 3'-PH (SEQ ID No 18)

### GENOTYPAGE sur LightCycler (par exemple cDNA de plaquettes)

cJAL2F GCACACAGAAACTATTCAGAGTC (SEQ ID No 19)
cJAK2S AGCAGCAAGTATGATGAGC (SEQ ID No 20)
cJAK2A CTAGTGATCCAAATTTTACAAACT (SEQ ID No 21)
cJAK2R GTTTAGCAACTTCAAGTTTCC (SEQ ID No 22)
Sensor wt GTCTCCACAGACACATACTCCATAA3'FL (SEQ ID No 23)
Anchor JAK2 5'-LC Red640AAAACCAAATGCTTGTGAGAAAGCT3'-PH (SEQ ID No 24)

### GENOTYPAGE par la technologie Taqman (par exemple sur ADN de cellules mononucléaires de Moelle Osseuse).

Reconnaissance par des sondes fluorescentes spécifiques d'allèle et ADN simple brin. Réaction de PCR
Sequence primer sens : AAGCTTTCTCACAAGCATTTGGTTT (SEQ ID No 25)
Sequence primer antisens : AGAAAGGCATTAGAAAGCCTGTAGTT (SEQ ID No 26)
Reporter 1 Sequence (VIC) : TCTCCACAGACACATAC (SEQ ID No 27)
Reporter 2 Sequence (FAM) : TCCACAGAAACATAC (SEQ ID No 28)

Dans un autre aspect, l'invention se rapporte à une méthode de diagnostic *in vitro* ou *ex vivo* permettant de détecter la présence ou l'absence de mutation JAK2 V617F dans un échantillon.

### Tests avec les acides nucléiques de l'invention

Dans un premier mode de réalisation, le variant G1849T (correspondant à la mutation JAK2 V617F) peut être détecté par analyse de la molécule d'acide nucléique du gène JAK2. Dans le cadre de la présente invention, on entend par « acide nucléique » un ARNm, l'ADN génomique ou l'ADNc dérivé de l'ARNm.

La présence ou l'absence de l'acide nucléique du variant G1849T peut être détecté par séquençage, amplification et/ou hybridation avec une sonde et des amorces spécifiques, telles que décrites ci-dessus : séquence dérivée des SEQ ID No 3 ou 4 et SEQ ID No 5 à 11 ou encore SEQ ID No 15 à 24.

L'invention propose donc une méthode *ex vivo* ou *in vitro* pour déterminer la présence ou l'absence du variant G1849T du gène JAK2 dans un échantillon d'un patient atteint de PV ou susceptible de développer une PV ou tout autre syndrome myéloprolifératif, notamment de l'éthrocytose, des thrombocytémies et myélofibroses, comprenant :
la détection de la présence ou de l'absence du variant G1849T du gène JAK2 dans un échantillon d'acide nucléique, du patient
caractérisée en ce que la présence du variant G1849T est une indication de PV ou de tout autre syndrome myéloprolifératif.

L'échantillon d'acide nucléique peut être obtenu à partir de n'importe quelle source cellulaire ou biopsie de tissu. Ces cellules doivent être d'origine hématopoïétique et peuvent être obtenus à partir de sang circulant, de tissu hématopoïétique ou tout fluide contaminé en cellules sanguines. L'ADN peut être extrait en utilisant toute méthode connue de l'état de la technique, telles que les méthodes décrites dans Sambrook et al. (1989). L'ARN peut également être isolé, par exemple à partir de tissus obtenus lors d'une biopsie, en utilisant des méthodes standard bien connues de l'homme du métier, telles que l'extraction par le guanidiumthiophénate-phénol-chlorophorme.

Le variant G1849T du gène JAK2 peut être détecté dans un échantillon d'ARN ou d'ADN, de préférence après amplification. Par exemple, l'ARN isolé peut être soumis à une transcription-réverse puis une amplification, telles qu'une réaction RT-PCR en utilisant les oligonucléotides spécifiques du site muté ou qui permettent l'amplification de la région contenant la mutation, par exemple l'exon 12 ou la séquence SEQ ID No 3 ou 4. L'expression « oligonucléotide » est utilisée ici pour désigner un acide nucléique d'au moins 10, de préférence entre 15 et 25 nucléotides, de préférence inférieur à 100 nucléotides, et qui est capable de s'hybrider à l'ADN génomique de JAK2, à l'ADNc ou encore à l'ARNm.

Les oligonucléotides de l'invention peuvent être marqués selon toute technique connue par l'homme du métier, tels que des marqueurs radioactifs, fluorescents ou enzymatiques. Un oligonucléotide marqué peut être utilisé comme sonde pour détecter la présence ou l'absence du variant G1849T du gène JAK2.

Ainsi, les sondes et amorces utiles selon l'invention sont celles qui s'hybrident spécifiquement à la région du gène JAK2 à proximité du nucléotide en position 1849 (numérotation à partir de l'ATG marquant le début de la transcription).

Dans la méthode explicitée ci-dessus, les acides nucléiques peuvent être amplifiés par PCR avant la détection de la variation allélique. Les méthodes pour la détection de la variation allélique sont décrites par exemple dans « Molecular Cloning - A Laboratory Manual » Second Edition, Sambrook, Fritsch and Maniatis (Cold Spring Harbor Laboratory, 1989) et Laboratory Protocols for Mutation Détection, Ed. par U. Landegren, Oxford University Press, 1996 et PCR, 2ème édition par Newton & Graham, BIOS Scientific Publishers Limited, 1997.

A ce titre, il est possible de combiner une étape d'amplification suivie par une étape de détection permettant de discriminer les échantillons en fonction de la présence ou de l'absence du variant recherché.

Différentes techniques adaptées à cet effet sont présentées dans EP 1 186 672, telles que le séquençage d'ADN, le séquençage par hybridation SSCP, DGGE, TGGE, l'analyse hétéroduplex, CMC, le clivage enzymatique de mésappariements, « hybridization based solid phase hybridization », les puces à ADN (DNA Chips), solution phase hybridization Taqman™ (US 5 210 015 et US 5 487 972) », ainsi que la technique RFLP.

La détection peut être réalisée en utilisant différentes méthodes alternatives : FRET, fluorescence quenching, fluorescence polarisée, chimiluminescence, électrochimiluminescence, radioactivité et méthode colorimétrique.

La méthode selon l'invention peut inclure extraire l'acide nucléique dudit échantillon.

Comme indiqué ci-dessus, l'échantillon utilisé peut être du sang ou tout autre fluide corporel ou tissu obtenu d'un individu. Après les étapes d'extraction et de purification de l'acide nucléique, l'amplification PCR au moyen des amorces décrites ci-dessus peut être utilisée pour améliorer la détection du signal.

Ainsi, la méthode selon l'invention peut comprendre l'étape d'amplification au moyen desdites amorces, suivie par une étape d'hybridation avec au moins une sonde, de préférence deux sondes qui s'hybrident en condition de forte stringence aux séquences correspondant à la région de la mutation G 1849T mentionnée ci-dessus et la détection du signal produit par les marqueurs desdites sondes.

Par exemple, l'invention vise plus particulièrement une méthode *in vitro* pour déterminer la présence ou l'absence du variant G1849T du gène JAK2 dans un échantillon d'un patient atteint de PV ou susceptible de développer une PV ou tout autre syndrome myéloprolifératif comprenant la détection de la présence ou de l'absence du variant G1849T du gène JAK2 dans ledit échantillon d'acide nucléique au moyen d'un ou plusieurs SNP (Single nucleotide polymorphism) spécifique de la mutation G1849T du gène JAK2, notamment les SEQ ID No 17, 18 ou encore 23 et 24;
caractérisée en ce que la présence du variant G1849T est une indication de PV ou de tout autre syndrome myéloprolifératif.

Cette détection au moyen de SNPs peut être mise en oeuvre par la Technologie TaqMan® permettant une discrimination allélique. Essentiellement, cette méthode consiste en la reconnaissance par des sondes fluorescentes spécifiques de l'allèle 1849 de JAK2 sur ADN simple brin et comprend une réaction PCR (avec une polymérase à activité 5' exonucléasique), la détection de l'émission de fluorescence spécifique d'allèle des SNP hybridés, la détermination du génotype par lecture de la fluorescence en point final (obtention d'une image montrant les clusters d'ADN muté homozygote, hétérozygote et normal).

### Détection de la protéine mutée JAK2 V617F

Selon un autre mode de réalisation, le variant peut être détecté directement au sein de la protéine JAK2.

A cet effet, l'invention vise une méthode *in vitro* pour détecter la présence ou l'absence du variant JAK2 V617F dans un échantillon d'un patient atteint ou susceptible de développer la PV ou tout autre syndrome myéloprolifératif, notamment de l'éthrocytose, des thrombocytémies et myélofibroses, comprenant :
la détection de la présence ou de l'absence du variant JAK2 V617F dans un échantillon du patient
caractérisée en ce que la présence dudit variant est une indication de la PV ou de tout autre syndrome myéloprolifératif.

Ledit variant JAK2 V617F peut être détecté par toute méthode appropriée connue de l'état de la technique.

Plus particulièrement, un échantillon, obtenu d'un individu, peut être mis en contact avec un anticorps spécifique du variant V617F de la protéine JAK2, par exemple un anticorps qui est capable de faire la distinction entre le variant V617F et la protéine JAK2 non mutée (et de tout autre protéine).

Les anticorps de la présente invention peuvent être des anticorps monoclonaux ou polyclonaux, simple chaîne ou double chaîne, des anticorps chimériques, humanisés ou des portions de molécules d'immunoglobuline comprenant les portions connues de l'état de la technique comme correspondant aux fragments de liaison à l'antigène [fragment humain, humain, F(ab')2 et F(v)].

Ces anticorps peuvent être immunoconjugués, par exemple avec une toxine ou un marqueur.

Les protocoles pour l'obtention d'anticorps polyclonaux sont bien connus de l'homme du métier. Typiquement, de tels anticorps peuvent être obtenus par l'administration du variant JAK2 V617F par injection souscutanée à des lapins blancs de Nouvelle-Zélande qui ont préalablement été préparés pour obtenir un sérum pré-immunitaire. Les antigènes peuvent être injectés jusqu'à 100 µl par site à 6 différents sites. Les lapins sont préparés deux semaines avant la première injection puis périodiquement stimulés par le même antigène, environ trois fois toutes les six semaines. Un échantillon de sérum est alors obtenu dix jours après chaque injection. Les anticorps polyclonaux sont ensuite purifiés du sérum par chromatographie d'affinité en utilisant la protéine JAK2 V617F pour capturer les anticorps.

Les anticorps monoclonaux sont préférés aux anticorps polyclonaux à cause de leur haute spécificité.
L'obtention de tels anticorps monoclonaux est à la portée de l'homme du métier, sachant que le variant JAK2 V617F présente une structure 3D différente de la protéine JAK2 sauvage. L'expression « anticorps monoclonal » signifie un anticorps qui est capable de reconnaître seulement un épitope d'un antigène.
Les anticorps monoclonaux peuvent être préparés par immunisation d'un mammifère, par exemple une souris, un rat, ou d'autres mammifères avec le variant JAK2 V617F purifié. Les cellules du mammifère immunisé produisant les anticorps sont isolées et fusionnées avec des cellules de myélomes ou d'hétéro-myélomes pour produire des cellules hybrides (hybridomes).

Les cellules d'hybridomes produisant l'anticorps monoclonal sont utilisées comme sources pour la production de l'anticorps. Les techniques de génération d'anticorps n'impliquant pas une immunisation sont également visées par l'invention. Il s'agit par exemple de la technologie « phage display ».

Les anticorps dirigés contre le variant JAK2 V617F peuvent dans certains cas présenter une réaction croisée avec la protéine JAK2 sauvage. Si tel est le cas, une sélection des anticorps spécifiques du variant V617F est nécessaire. A ce titre, on peut utiliser par exemple une chromatographie d'affinité avec la protéine JAK2 sauvage pour capturer les anticorps qui présenterait une réaction croisée avec JAK2 sauvage.

Ainsi, l'invention vise un anticorps monoclonal reconnaissant spécifiquement le variant JAK2 V617F ainsi que les lignées d'hybridomes le produisant.
L'invention porte également sur un test ELISA au moyen dudit anticorps pour détecter la présence ou l'absence du variant JAK2 V617F dans un échantillon.

Une alternative à l'utilisation des anticorps peut consister par exemple à la préparation et l'identification d'haptamères qui sont des classes de molécules permettant une reconnaissance moléculaire spécifique.
Les haptamères sont des oligonucléotides ou des oligopeptides qui peuvent virtuellement reconnaître n'importe quelle classe de molécules cibles avec une haute affinité et spécificité.

### Kits

Dans un autre aspect, l'invention concerne des kits pour déterminer si un patient est atteint de la polyglobulie de Vaquez ou de tout autre syndrome myéloprolifératif impliquant le variant JAK2 V617F.

Le kit de l'invention peut comprendre une ou plusieurs sondes ou amorces telles que définies ci-dessus pour la détection spécifique de la présence ou de l'absence de la mutation G1849T dans le gène JAK2.

Le kit peut également comprendre une polymérase thermorésistante pour l'amplification PCR, une ou plusieurs solutions pour l'amplification et/ou l'étape d'hybridation, ainsi que tout réactif permettant la détection du marqueur.

Dans un autre mode de réalisation, le kit comprend un anticorps tel que défini ci-dessus.

Les kits de l'invention peuvent contenir en outre tout réactif adapté à l'hybridation ou à la réaction immunologique sur support solide.

La méthode et le kit de détection sont avantageusement pratiqués pour la sous-population de patients présentant un taux d'hématocrites supérieur à 51 %. La méthode et le kit de détection sont également avantageusement pratiqués pour la sous-population de patients présentant un taux de plaquettes supérieur à 450 000.

### siRNA de l'invention

Dans un quatrième aspect, l'invention porte également sur des siRNA permettant de diminuer de plus de 50%, 75%, 90% ou 95% ou encore de plus de 99% l'expression de JAK2 mutée en position 617, en particulier de JAK2 V617F. Ces siRNA peuvent être injectés dans les cellules ou tissus par lipofection, transduction, ou électroporation. Ils permettent de détruire spécifiquement les ARNm codant pour JAK2 V617F impliquant de nombreuses applications thérapeutiques possibles, notamment le traitement de la polyglobulie de Vaquez.

Des siRNA sont décrits dans US 60/068562 (CARNEGIE). L'ARN est caractérisé en ce qu'il possède une région avec une structure double brin (db). L'inhibition est spécifique de la séquence cible, la séquence nucléotidique d'un brin de la région db de l'ARN comprenant au moins 25 bases et étant identique à la portion du gène cible. La séquence nucléotidique de l'autre brin de la région db de l'ARN est complémentaire à celle du premier brin et à la portion du gène cible. Par ailleurs, la demande WO 02/44 321 (MIT/MAX PLANCK INSTITUTE) décrit un ARN double brin (ou des oligonucléotides de même nature synthétisés chimiquement) dont chaque brin a une longueur de 19 à 25 nucléotides et qui est capable d'inhiber spécifiquement l'expression post transcriptionnelle d'un gène cible par un processus d'interférence ARN afin de déterminer la fonction d'un gène et afin de moduler cette fonction dans une cellule ou un organisme. Enfin, WO 00/44895 (RIBOPHARMA) porte sur un procédé d'inhibition de l'expression d'un gène cible donné dans une cellule eucaryote *in vitro,* dans lequel un ARNdb formé de deux ARN simples brins séparés est introduit dans la cellule, un brin de l'ARNdb présentant une région complémentaire du gène cible caractérisé en ce que la région complémentaire présente moins de 25 paires de nucléotides successives. L'homme du métier pourra se référer à l'enseignement contenu dans ces documents pour la préparation des siRNA de la présente invention.

Plus spécifiquement, l'invention porte sur des ARN double brin d'environ 15 à 30 nucléotides, 19 à 25 nucléotides, ou de préférence environ 19 nucléotides de long complémentaires (brin 1) et identiques (brin 2) à la séquence SEQ ID No 3 comprenant la mutation G1849T. Ces siRNA de l'invention peuvent comporter en outre un dinucléotide TT ou UU à l'extrémité 3'.
De nombreux programmes sont disponibles pour le design des siRNA de l'invention:
- «siSearch Program» à http://sonnhammer.cgb.ki.se/siSearch/siSearch_1.6.html (« Improved and automated prediction of effective siRNA », Chalk AM, Wahlesdelt C, and Sonnhammer ELL, Biochemical and Biophysical Research Communications, 2004).
- « SiDirect » à http://design.mai.jp/sidirect/index.php (Direct: highly effective, target-specific siRNA design software for mammalian RNA interference, Yuki Naito et al, Nucleic Acids Res, Vol. 32, No. Web Server issue © Oxford University Press 2004).
- « siRNA Target Finder » de Ambion à l'adresse
   http://www.ambion.com/techlib/misc/siRNA_tools.html
- Le « siRNA design tool » du Whitehead Institute of Biomedical Research au MIT à l'adresse http://jura.wi.mit.edu/pubint/http://iona.wi.mit.edu/siRNAext/
D'autres programmes sont listés à :
http://web.mit.edu/mmcmanus/www/home 1.2files/siRNAs.htm,
notamment http://athena.bioc.uvic.ca/cgi-bin/emboss.pl?_action=input&_app=sima
Par exemple, pour la séquence TATGGAGTATGTT¹⁸⁴⁹**TCTGTGGAGA** (SEQ ID No 12), le siRNA sens est UGGAGUAUGUUUCUGUGGAdTdT (SEQ ID No 13) et le siRNA antisens est UCCACAGAAACAUACUCCAdTdT (SEQ ID No 14).
Dans un mode de réalisation particulier, les siRNA de l'invention décrits ci-dessus sont testés et sélectionnés pour leur capacité à réduire, voir bloquer spécifiquement l'expression de JAK2 V617F en affectant le moins possible l'expression de JAK2 sauvage. Par exemple, l'invention vise des siRNA permettant une diminution supérieure à 80%, 90%, 95% ou 99 % de l'expression de JAK2 V617F et aucune diminution ou une diminution inférieure à 50%, 25%, 15%, 10% ou 5% ou encore inférieure à 1% de JAK2 sauvage.

Par exemple, les siRNA de l'invention peuvent être sélectionnés dans le groupe constitué de:

Dans un autre mode de réalisation, l'invention concerne une molécule ddRNAi telle que décrite de manière générique dans la demande WO 01/70949 (Benitec) mais ciblant spécifiquement JAK2 V617F. Le ddRNAi de l'invention permet l'extinction de la séquence codant pour JAK2 V617F et comprend (i) une séquence identique à la SEQ ID No 3, 4 ou 11 ; (ii) une séquence complémentaire à la séquence défini en (i) ; (iii) un intron séparant lesdites séquences (i) et (ii) ; l'introduction de cette construction dans une cellule ou un tissu produisant un ARN capable d'altérer l'expression de JAK2 V617F.
L'invention porte également sur un animal non humain génétiquement modifié comprenant une ou plusieurs cellules contenant une construction génétique capable de bloquer, de retarder ou de réduire l'expression de JAK2 V617F dans l'animal. La méthode de production d'un tel animal génétiquement modifié est présentée dans WO 04/022748 (Benitec).

### Méthodes de criblage

Dans un cinquième aspect, l'invention a pour objet une méthode de criblage d'inhibiteurs spécifiques de JAK2 V617F.

Par « inhibiteurs spécifiques », on entend désigner des composés présentant un ratio IC50 sur JAK2 / IC50 sur JAK2 V617F supérieur à 5, 10, 25, ou encore 50. Par exemple, le composé possède une IC50 JAK2 V617F inférieure à 1 µM, de préférence 100 nM alors qu'il possède une IC50 JAK2 supérieure à 5 µM ou 10 µM.

Cette méthode peut être mise en oeuvre à l'aide de la protéine de l'invention, d'une fraction membranaire comprenant ladite protéine, d'une cellule exprimant ladite protéine ou encore d'un animal transgénique non humain tel que décrit plus haut.

Ainsi, l'invention porte sur un test permettant de déterminer l'inhibition spécifique de JAK2 V617F par un ou plusieurs composés comprenant les étapes consistant à mettre en contact un ou plusieurs composés avec la protéine JAK2 V617F décrite ci-dessus, une fraction membranaire comprenant JAK2 V617F ou encore une cellule exprimant JAK2 V617F décrite ci-dessus dans des conditions appropriées pour une fixation et une détection de la fixation spécifique et/ou de l'inhibition de JAK2 V617F.
Ce procédé peut comprendre en outre une mesure de la fixation sur JAK2 sauvage.
Ce procédé peut également consister en une succession de tests de plusieurs molécules et comprendre une étape de sélection des molécules présentant une IC50 pour JAK2 V617F inférieure à 1 µM, de préférence 100 nM.
Ce procédé peut comprendre en outre une étape de sélection négative des molécules mentionnées ci-dessus qui possèdent une IC50 pour JAK2 inférieure à 5 µM ou 1 µM.

L'invention porte sur un criblage *in vitro* tel que décrit ci-dessus dans lequel on détermine par immunoprécipitation l'inhibition de la phosphorylation de JAK2 V617F.

L'invention porte également sur un criblage in vitro sur des cellules progénitrices CD34+ JAK2 V617F qui sont capables de se différencier sans érythropoiétine (Epo). De telles cellules isolées de patients atteints de la polyglobulie de Vaquez. Les cellules CD34+ JAK2 V617F peuvent être mises en culture dans un milieu comprenant du SCF et IL-3. Les composés sont rajoutés dans le milieu de culture et on détermine la capacité des cellules à proliférer et à se différencier en cellules 36+/GPA-. On sélectionne les composés pour lesquels une diminution du nombre de clones 36+/GPA- est observée. Ainsi, l'invention porte sur la méthode de criblage ci-dessus au moyen de cellules primaires progénitrices CD34+ JAK2 V617F qui sont capables de se différencier sans érythropoïétine (Epo) ou sur des lignées cellulaires devenues facteur indépendantes par introduction du variant JAK2 V617F. Le même type d'essai peut être réalisé sur des cultures de moelle de type CFU-E en milieu semi-solide et testé directeent le composé sur l'inhibition de la croissance sponténée des colonies.
On peut également utiliser toute lignée cellulaire de mammifère décrite ci-dessus exprimant JAK V617F recombinante.

L'invention porte également sur une méthode pour l'identification de candidats médicaments comprenant les étapes consistant à administrer des composés à un animal transgénique non humain exprimant JAK2 V617F tel que décrit plus haut, ledit animal reproduisant la polyglobulie de vaquez et/ou étant atteint d'un syndrome myéloprolifératif associé à la présence de JAK2 V617F, à déterminer l'effet du composé et sélectionner les candidats médicaments pour lesquels on observe une diminution ou un blocage de la prolifération et de la différenciation spontanées des érythroblastes de polyglobulie de vaquez ou une diminution de la prolifération cellulaire associé à la présence de JAK2 V617F.

Plus particulièrement, cette méthode est réalisée avec une souris K-in JAK2 V617F ou un rat K-in JAK2 V617F tel que décrit ci-dessus.

Parmi ces composés, on peut citer par exemple les siRNA ciblant l'exon 12 muté de JAK2 mentionnés ci-dessus, en particulier des siRNA ciblant la séquence SEQ ID No 3, 4 ou encore la séquence SEQ ID No 11 comprenant le nucléotide muté t¹⁸⁴⁹.

Un autre aspect de l'invention porte sur l'utilisation desdits SiRNA ou ddRNAi décrits ci-dessus, et des composés inhibant spécifiquement JAK2 V617F pour la fabrication d'un médicament. Ledit médicament est notamment destiné au traitement des cancer du sang, en particulier les syndromes myéloprolifératifs comprenant la Polyglobulie de Vaquez, la thrombocythénie essentielle, la splénomégalie myéloïde ou myélofibrose primitive et la leucémie myéloïde chronique (LMC). Ledit médicament est également destiné au traitement d'autres hémopathies malignes; associées à la mutation JAK2 V617F, et le cas échéant des tumeurs solides, des carcinomes, des mélanomes et des neuroblastomes, qui expriment JAK2 V617F.

Pour la suite de la description et pour les exemples, on se référera aux figures dont la légende est indiquée ci-dessous.

### Légendes

### Figure 1 : Découverte du rôle clé de JAK2 dans la PV

A l'état basal, JAK2 est fixé sur la box 1 à l'état non phosphorylé. La liaison à l'EPO modifie la conformation du récepteur et permet la transphosphorylation de JAK2 qui en retour phosphoryle les résidus intracytoplasmiques de EPO-R et recrute ainsi les différents effecteurs positifs (->) ou négatifs ( -| ) de la transduction du signal.

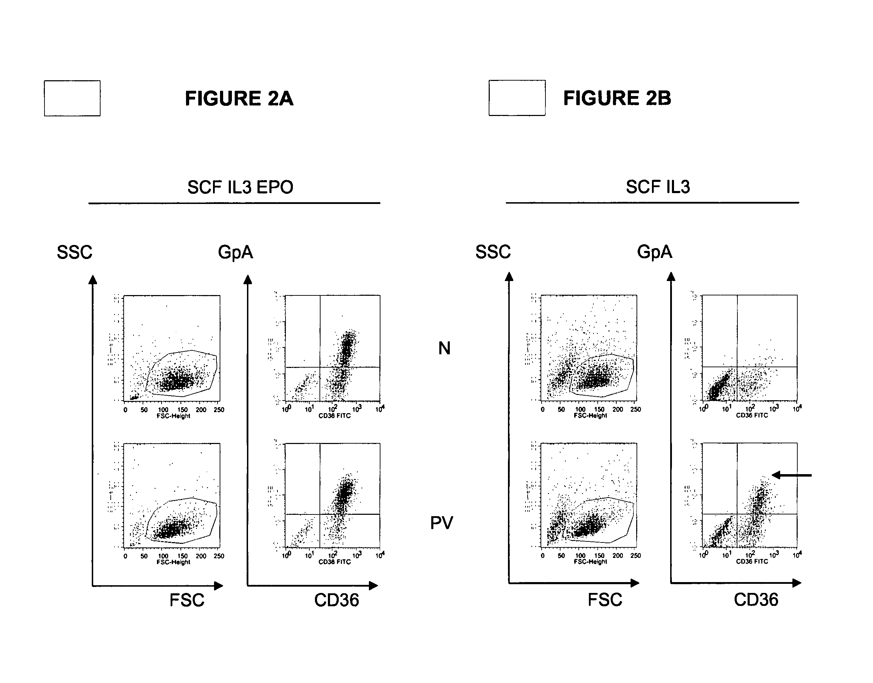

**Figure 2 : Mise au point d'un modèle de culture de progéniteurs CD34+ de PV qui peuvent se différencier sans érythropoïétinc.**
2A- culture avec Epo, SCF et IL-3
2B- culture sans Epo.

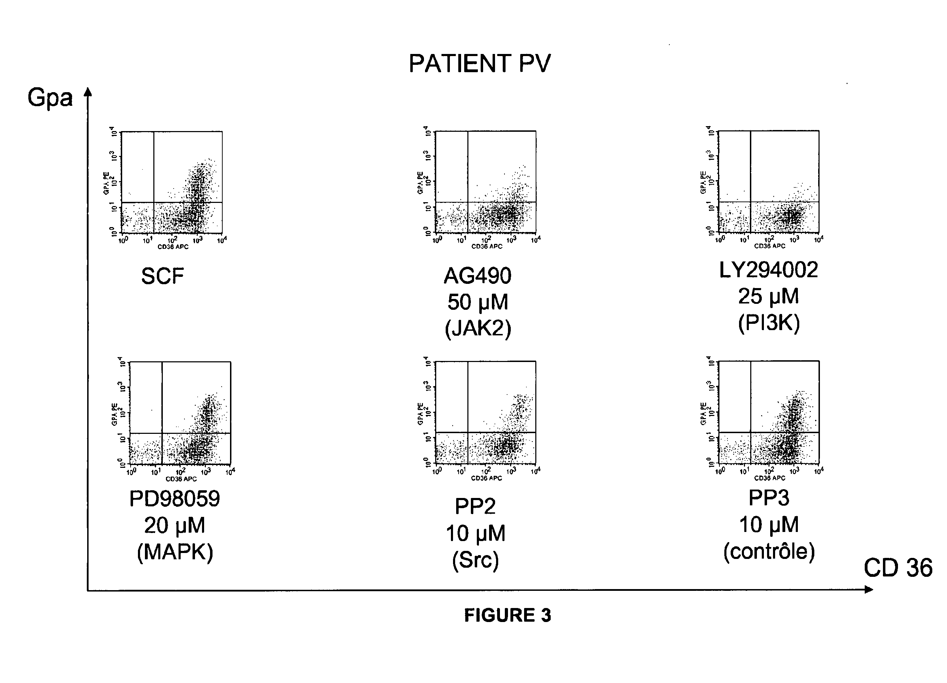

**Figure 3 : L'inhibition des voies JAK-STAT, Pi3-K et Src kinase empêche la différenciation érythroïde spontanée.**

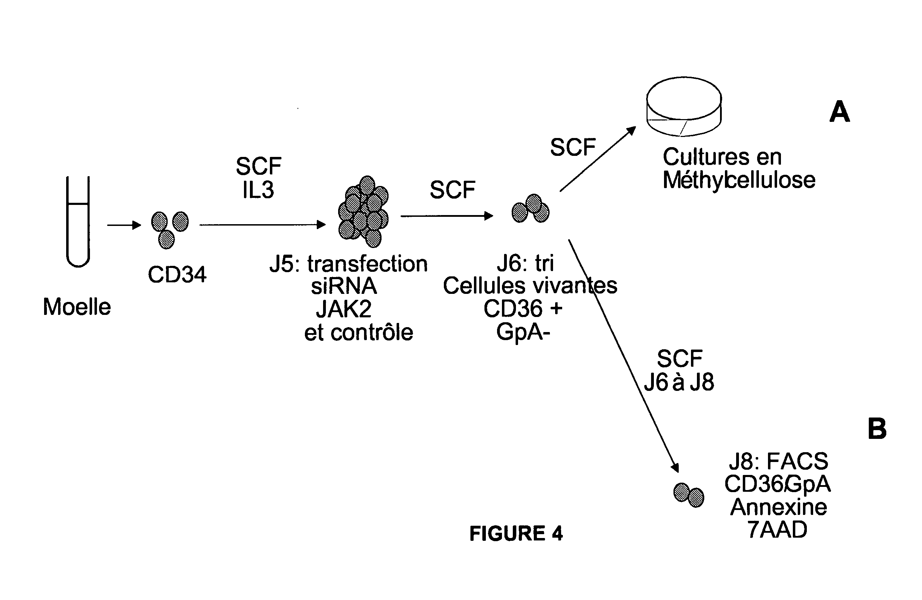

**Figure 4 : Protocole d'inhibition de JAK2 dans les progéniteurs de PV.**

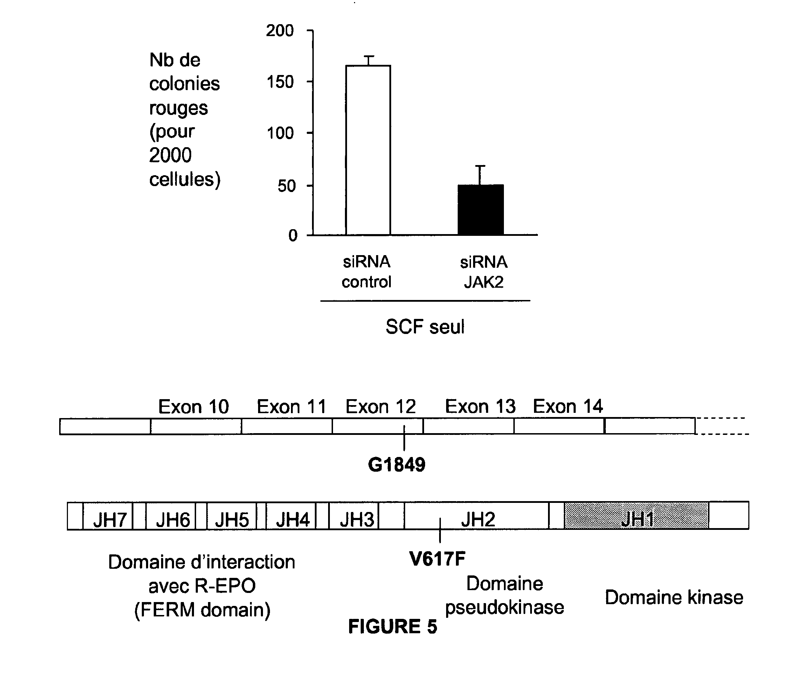

**Figure 5 : Résultats de l'inhibition de JAK2 dans les progéniteurs de PV.**
5A- Diminution de la capacité de clonage des 36+/gpa- Culture J1-J6 en SCF-IL3, électroporation J5 tri J6 (morpho/36+/gpa-).
Méthyl SCF seul. Compte à J13 (J7 post tri)
5B- Structure de JAK2 avec la mutation V617F (exon 12)
**Figure 6 : Analyses de génotypage des SNP pour détecter la mutation JAK2 V617F dans l'ADN génomique avec les technologies LightCycler® et TaqMan®**
**A et B** : Détection de la mutation par la courbe d'analyse de fusion de LightCycler® avec les sondes d'hybridation FRET. A : Des expériences avec diverses dilutions d'ADN HEL dans de l'ADN TF-1 sont représentées. Le pic JAK2 V617F (57°) est encore détectable à une dilution de 1%. B : Des résultats provenant d'échantillons de patients représentatifs sont représentés (#1 : homozygote ; #2 : hétérozygote ; #3 : faible ; #4 : non muté).
**C** : Détection de la mutation par amplification spécifique d'allèle TaqMan®. Des expériences de dilution d'ADN HEL (HEL de 100 à 1% : carrés vides ; cellules TF-1 : cercles vides) et quelques échantillons de patients représentatifs sont représentés (croix noires). #a : patients homozygotes ; #b : patients hétérozygotes ; #c : patient faible ; #d : patients non mutés.

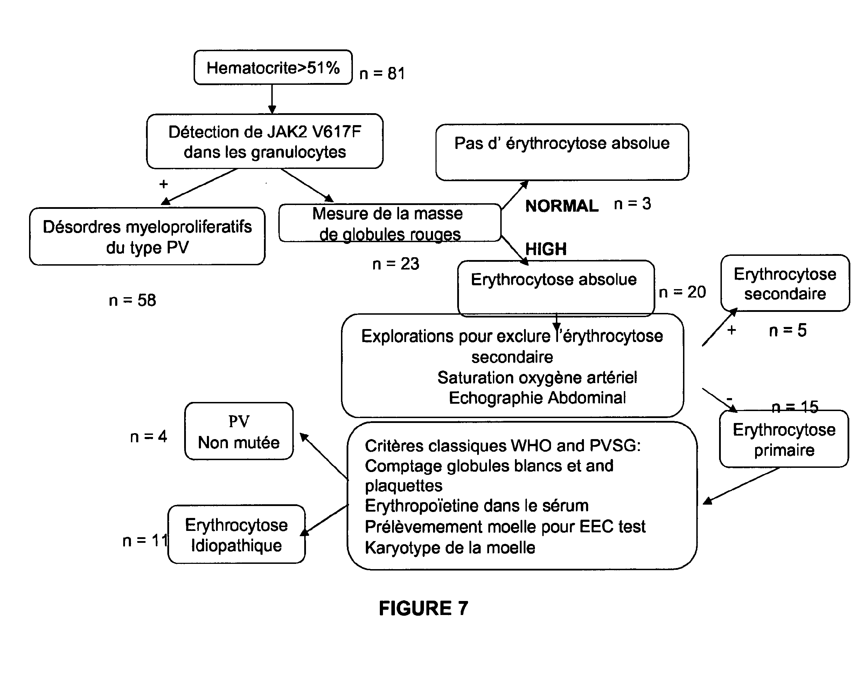

**Figure 7 : Fiche de diagnostic proposée pour le diagnostic d'une érythrocytose (c'est-à-dire un taux d'hématocrites supérieur à 51%).**
Le nombre de patients concernés à chaque étape de la fiche est écrit à côté de chaque article (n), seuls les patients ayant eu toutes les données cliniques étant listés ici (n = 81). La détection de JAK2 V617F en tant que test de diagnostic en première intention aurait évité à 58/81 patients d'avoir d'autres investigations pour diagnostiquer un syndrome myéloprolifératif de type PV.

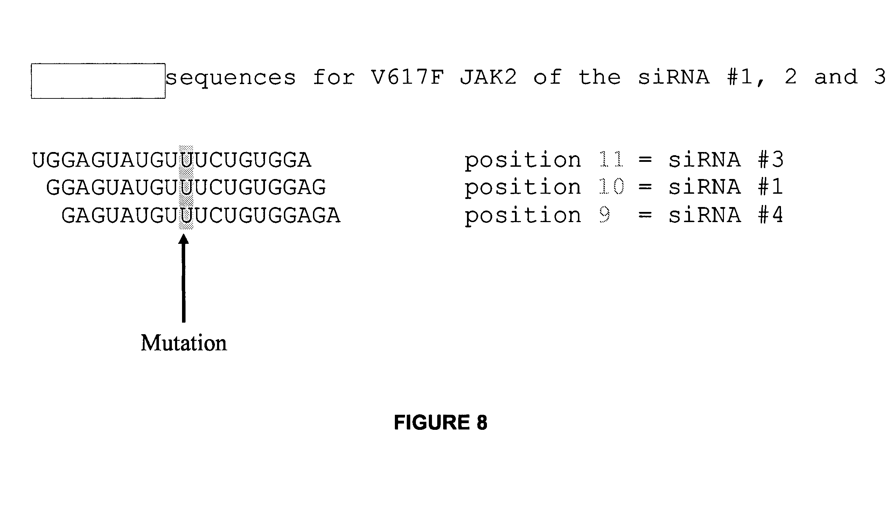

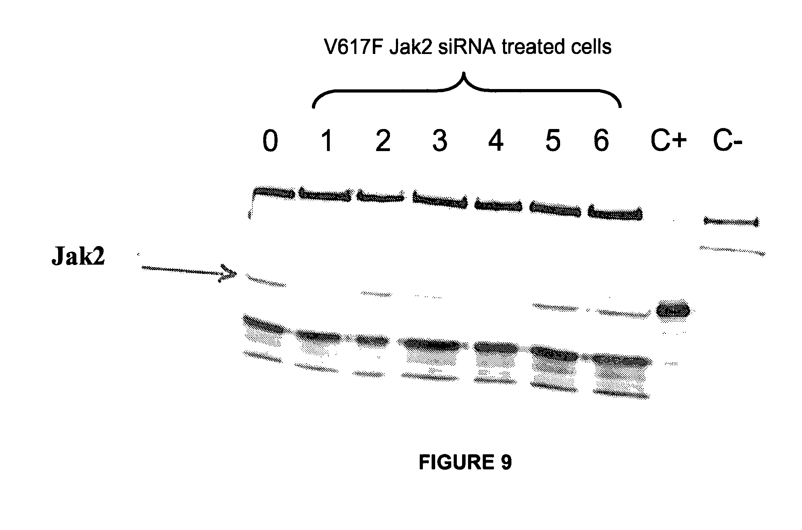

**Figures 8 et 9 : L'expression de V617F Jak2 dans des cellules HEL est diminuée 24 heures après le traitement au siRNA spécifique de JAK2 V617F Jak2 (siRNA #1, 3 et 4).**
0 à 6 : Cellules HEL traitées (1 à 6) ou non traitées (0) avec les siRNA V617F Jak2.
C+ : Cellules 293HEK transfectées avec le vecteur V617F Jak2 RV.
C- : 293HEK.

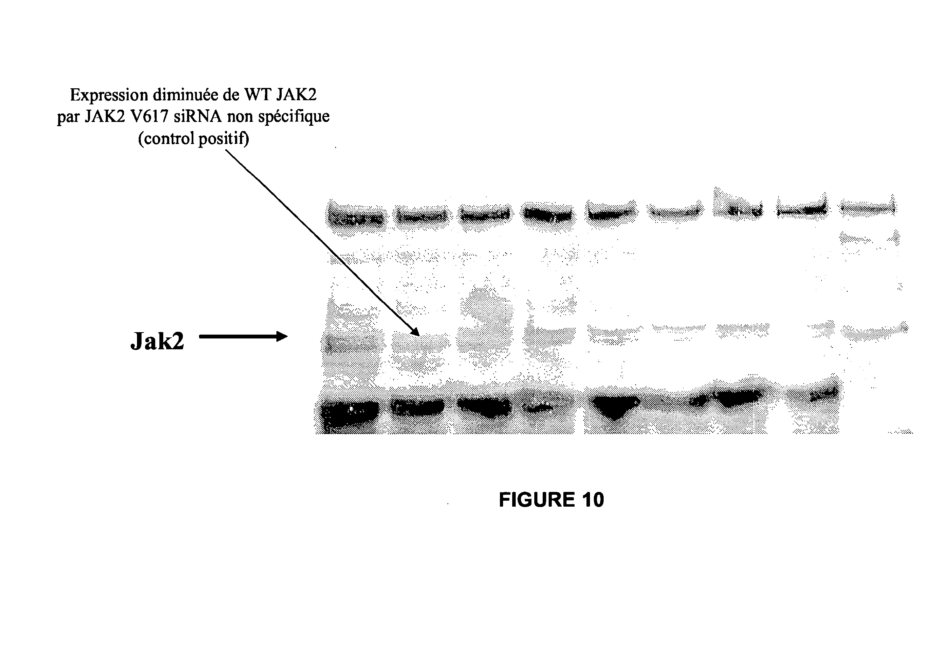

**Figure 10 : Le niveau d'expression de WT Jak2 dans des cellules K562 n'est pas changé 24 heures après un traitement au siRNA spécifique de Jak2 V617.**
Je : Cellules K562 traitées avec les siRNA WT Jak2.
0 à 6 : Cellules K562 traitées avec les siRNA V617F Jak2.
C- : 293HEK (pas d'expression de JAK2).

### Exemple 1: Identification de la mutation JAK2 V617F dans 39/43 patients.

La mise au point d'un modèle de culture cellulaire des progéniteurs pathologiques et l'utilisation d'inhibiteurs biochimiques nous a permis d'établir que les voies JAK2-STAT5, P13 kinase, et Src kinases sont nécessaires à la différenciation indépendante de l'Epo des progéniteurs de PV (Ugo et al, 2004). Ces résultats nous ont conforté dans notre hypothèse selon laquelle la lésion moléculaire primaire à l'origine de la PV devait être une anomalie d'une protéine clef aboutissant à la dérégulation d'une voie de signalisation, comme une mutation d'une tyrosine kinase lui conférant une activité constitutive. Néanmoins, c'est l'étude de 43 patients atteints de la polyglobulie de Vaquez qui a permis d'identifier le rôle clé de la protéine JAK2, qui est la protéine située le plus en amont dans ces différentes voies de signalisation, et qui est commune aux voies de signalisation des récepteurs aux cytokines pour lesquelles des anomalies de réponse ont été décrites dans la PV. Nous avons étudié l'implication de la protéine kinase JAK2 dans la physiopathologie de la PV par 3 abords complémentaires :
- un abord fonctionnel (inhibition de JAK2 dans les cellules de PV par ARN interférence),
- un abord génomique (séquençage des 23 exons du gène), et
- un abord biochimique à la recherche d'une anomalie de phosphorylation de JAK2 à l'origine d'une activation constitutive.
Le matériel biologique utilisé provient de patients consentants atteints de polyglobulie et correspond aux résidus de prélèvements réalisés à visée diagnostique adressés au Laboratoire Central d'Hématologie de l'Hotel Dieu, et à des saignées thérapeutiques.

### 1.1 Etude fonctionnelle

L'étude de la fonction de JAK2 dans les érythroblastes de patients atteints de polyglobulie de Vaquez a été réalisée en transduisant par électroporation des érythroblastes de PV avec un siRNA spécifique de la séquence JAK2 (AMBION, Huntingdon, Angleterre) reconnaissant comme cible une séquence située sur l'exon 15 de l'ARNm de JAK2. Nous avons montré que l'inhibition de JAK2 diminuait fortement la capacité de clonage et la différenciation « spontanée » des progéniteurs de PV en l'absence d'Epo. Les progéniteurs érythroblastiques normaux transfectés avec le siRNA JAK2 ont un potentiel clonogénique diminué de 70% par rapport au siRNA contrôle ce qui confirme l'efficacité de la transfection avec le siRNA JAK2. Dans la PV, les effets de l'inhibition de JAK2 dans les progéniteurs érythroblastiques ont été étudiés dans un modèle de culture sans Epo, permettant d'étudier les cellules du clone malin. Nous avons comparé le potentiel clonogénique, l'apoptose et la différenciation des progéniteurs érythroblastiques de PV après transfection par un siRNA JAK2 comparé à un siRNA contrôle. L'étude du potentiel clonogénique des progéniteurs de PV cultivés sans Epo montre une très nette diminution du nombre de colonies après transfection du siRNA JAK2 par rapport au siRNA contrôle. L'étude en cytométrie de flux de l'apoptose de ces cellules montre une augmentation significative de l'apoptose dans les cellules transfectées par le siRNA JAK2 comparées au siRNA non relevant (70 versus 53%). L'étude des effets du siRNA JAK2 sur la différenciation (acquisition de la Glycophorine A détectée en cytométrie de flux), ne montre qu'une légère différence entre les progéniteurs transfectés par le siRNA JAK2 versus le siRNA contrôle.

Les résultats des études cellulaires ont donc montré que JAK2 était nécessaire à la différenciation indépendante de l'Epo des progéniteurs érythroïdes de PV. Les premiers résultats des études biochimiques (immunoprécipitation) montrent une phosphorylation de JAK2 plus prolongée après déprivation en cytokines des progéniteurs érythroïdes de PV par rapport à des cellules normales.

### 1.2 Etude génomique de JAK2

La PCR sur les 23 exons a été mise au point sur un sujet normal à partir d'ADN génomique. Puis nous avons étudié 3 patients atteints de PV après avoir extrait l'ADN génomique de cellules érythroïdes obtenus *in vitro* après culture cellulaire.
Nous avons identifié une mutation ponctuelle située dans l'exon 12 de JAK2, présente chez 2 des 3 patients testés. Cette mutation touche la base n° 1849 de la séquence codante [numérotation débutant à l'ATG], GenBank NM_004972) transforme le codon 617 de la protéine JAK2 (V617F).
- Codon 617 normal : gtc code pour une Valine (V)
- Codon 617 muté : ttc code pour une Phénylalanine (F)
Nous avons vérifié grâce aux bases de données publiées sur Internet qu'il ne s'agissait pas d'un polymorphisme connu.
Nous avons ensuite élargi la cohorte. A ce jour, la mutation a été retrouvée chez 39 patients atteints de PV sur les 43 cas testés. Aucun témoin (15 cas testés) ou patient atteint de polyglobulie secondaire (18 cas testés) n'a été trouvé porteur de la mutation.

### Résultats du séquençage chez les patients

- 39 mutés / 43 PV testés (90%)
- 2/3 hétérozygotes
- 13/39 « homozygotes » soit 30% des cas (même proportion que la perte d'hétérozygotie en 9p)

### Contrôles

- 0 cas mutés sur 33 témoins testés
   - Dont 15 sujets normaux
   - Et 18 polyglobulies secondaires (pas de colonies spontanées)

La découverte de cette anomalie de JAK2 explique l'hypersensibilité à de nombreux facteurs de croissance impliquée dans la PV (Epo, TPO, IL-3, IL-6, GM-CSF, insuline). En effet, JAK2 est une protéine impliquée dans les voies de signalisation des récepteurs de ces cytokines.

De plus, l'association de JAK2 avec R-Epo est singulière en cela que JAK2 est fixée à R-Epo de façon constitutive : l'association JAK2/R-Epo initiée dans l'appareil de Golgi, est nécessaire au processing de R-Epo à la membrane des érythroblastes. Une anomalie de JAK2 à l'origine de modifications de l'association de JAK2 avec R-Epo pourrait donc expliquer la prédominance de l'hyperplasie médullaire sur la lignée érythroblastique, alors que cette protéine est impliquée dans de nombreuses voies de signalisation. Par ailleurs, Moliterno et coll (Moliterno et al., 1998; Moliterno et Spivak, 1999) ont mis en évidence un défaut d'expression membranaire de mpl, lié à un défaut de glycosylation. Il est possible que JAK2 soit, par analogie avec R-Epo, nécessaire au processing de c-mpl. L'anomalie de JAK2 pourrait alors expliquer le défaut d'expression membranaire de c-mpl, retrouvé dans la PV.

JAK2 se lie à R-Epo sur son domaine proximal au niveau d'un domaine très conservé, la Box2. En l'absence de stimulation par l'Epo, JAK2 est constitutivement fixée à R-Epo, mais sous forme non phosphorylée donc non active. Après stimulation du récepteur par l'Epo, les deux molécules JAK2 se phosphorylent, puis phosphorylent R-Epo, ce qui permet le recrutement puis la phosphorylation d'autres protéines impliquées dans la transduction du signal, comme les protéines STAT5, Grb2, PI3K. La protéine JAK2 présente, comme toutes les JAKs, un domaine kinase fonctionnel (JH1), un domaine pseudo-kinase sans activité tyrosine kinase (JH2), et plusieurs domaines conservés (JH3-JH7), caractéristiques des membres de la famille des JAKs. Le domaine JH2 semble impliqué dans la régulation de l'activité tyrosine kinase de JAK2. D'après les données disponibles de cartographie protéique de JAK2 (Lindauer, 2001), l'acide aminé 617 est situé dans ce domaine JH2, et d'après des études de modélisation, dans une région particulièrement importante pour la régulation de l'activité kinase.

Au delà de l'intérêt physiopathologique de cette découverte (compréhension des mécanismes d'indépendance aux cytokines, démembrement des différents SMP), la mise en évidence de la mutation chez un patient offre pour la première fois un test permettant d'affirmer le diagnostic. Dans une démarche médicale diagnostique, la recherche de la mutation de JAK2 pourra se faire sur les polynucléaires sanguins, qui appartiennent au clone malin.
L'invention offre également la mise au point d'un traitement spécifique, de type inhibiteur de kinase spécifique de la protéine mutée, ou thérapie génique.

### Exemple 2 : Détection du mutant JAK2 V617F pour le diagnostic de l'érythrocytose en première intention

### 2.1 PATIENTS, MATERIAUX ET METHODES

### Comparaison du séquençage et de deux techniques de génotypage de SNP pour la détection de JAK2 V617F

### Cellules du patient

On a analysé 119 échantillons à suspicion de MPD (c'est-à-dire d'érythrocytose, de thrombocytose, d'hyperleucocytose) : 58 échantillons sanguins ont été recueillis en perspective et 61 échantillons d'archive de moelle osseuse ont été analysés rétrospectivement.
On a isolé les granulocytes périphériques en utilisant une méthode de gradient de densité selon les instructions du fabriquant (Eurobio, France). On a isolé les cellules mononucléaires de la moelle osseuse par l'utilisation d'une centrifugation au gradient de Ficoll. L'ADN génomique a été extrait avec des procédures standard. Pour établir la sensibilité des technologies LightCycler® et TaqMan®, l'ADN provenant d'un échantillon homozygote avec l'allèle de type sauvage résiduel minimal, a été dilué en série dans de l'ADN normal.

### Lignées cellulaires

On a utilisé des dilutions d'ADN en série (1, 0,5, 0,1, 0,05, 0,01) de la lignée cellulaire d'érythroleucémie humaine (HEL) mutée de façon homozygote (JAK2 V617F), dans de l'ADN de lignée cellulaire TF-1 (non mutée) en tant que contrôles positifs standard. Les lignées cellulaires ont poussé dans du milieu MEM-alpha (Invitrogen) enrichi avec du sérum foetal de veau.

### Détection de la mutation par analyse de la courbe de fusion de LightCycler® avec des sondes d'hybridation FRET

On a conçu des amorces et des sondes pour amplifier et s'hybrider à la séquence cible de l'exon 12 de JAK2. La position du site de mutation (1849G/T) était couverte par une sonde capteur donneuse marquée à la fluorescéine en 3' et la sonde d'ancrage accepteuse adjacente marquée avec du LightCycler® Red 640 (LCRed640) à son extrémité 5', et son extrémité 3' a été phosphorylée pour éviter l'extension. L'amplification et l'analyse de la courbe de fusion ont été mises en oeuvre sur l'instrument de LightCycler® (Roche Diagnostics, Meylan, France). Le volume réactionnel final était de 20 µl en utilisant 10 ng d'ADN, 14 µl de mélange LightCycler FastStart DNA Master, 3 mM de MgCl₂, 0,2 µM d'amorces, 0,075 µM de chaque sonde. En bref, les échantillons ont été chauffés à 95°C pendant 10 minutes et une amplification par PCR de 45 cycles (10 secondes à 95°C, 10 secondes à 53°C, 15 secondes à 72°C) a été suivie par une étape de dénaturation à 95°C pendant 10 secondes, deux étapes d'hybridation à 63°C et 45°C pendant 30 secondes chacune et une courbe de fusion se situant dans le domaine compris entre 45 et 70°C (0,1 °C/sec). L'analyse sur le programme LightCycler® a été réalisée par le tracé de la courbe de la dérivée de la fluorescence par rapport à la température [2(dF12/F11)/dT) versus T]. Les pics mutés et WT ont été respectivement observés à 57 et 63°C.

### Détection de la mutation par amplification spécifique d'un allèle par TaqMan®

Deux amorces ont été conçues pour amplifier un produit de 92 pb englobant le SNP à la position 1849. Deux sondes MGB fluorogéniques ont été conçues avec différents colorants fluorescents, un ciblé vers l'allèle WT et un ciblé vers celui muté. On a réalisé le génotypage dans des plaques à 96 puits, en utilisant la méthode basée sur la TaqMan® PCR. Le volume réactionnel final était de 12,5 µl en utilisant 10 ng d'ADN génomique, 6,25 µl de TaqMan® Universal Master Mix et 0,31 µl de 40X Assays-on-Demand SNP Genotyping Assay Mix (Applied Biosystems). La plaque a été chauffée à 95°C pendant 10 minutes, suivi par 40 cycles de dénaturation à 92°C pendant 15 secondes et un appariement/extension à 60°C pendant 1 minute. Le thermocyclage a été réalisé sur le 7500 Real Time PCR System (Applied Biosystems). L'analyse a été effectuée avec le programme SDS version 1.3. Le génotypage de la discrimination allélique en point final a été réalisé par inspection visuelle d'une courbe de la fluorescence (Rn) provenant de la sonde WT contre la Rn de la JAK2 mutée générée à partir de la lecture de la fluorescence post-PCR.

### Patients avec une érythrocytose et collecte d'échantillons

Nous avons évalué 88 patients avec des taux d'hématocrites supérieurs à 51%, au moment du diagnostic, avant un quelconque traitement, et étudié la présence des critères WHO et PVSG. La valeur de 51% a été retenue pour l'extrémité supérieure du domaine normal pour le taux de l'hématocrite (Pearson TC et al., A Polycythemia Vera Updated : Diagnosis, Pathobiology, and Treatment. Hematology (Am. Soc. Hematol. Educ. Program.) 2000 : 51 à 68). Les cellules de la moelle osseuse ont été recueillies pour des dosages clonogéniques et les cellules excédentaires ont été recueillies pour l'extraction d'ADN. On a mesuré l'érythropoïétine sérique (Epo) dans différents laboratoires, et elle est par conséquent, rapportée comme étant basse quand elle est en dessous de la valeur inférieure du domaine normal de chaque laboratoire, normale ou élevée. Les granulocytes périphériques provenant des mêmes patients ont été purifiés comme décrit précédemment, les échantillons sanguins de chaque temps étaient disponibles. Les échantillons de moelle osseuse et de sang ont été recueillis après qu'on ait obtenu un consentement éclairé.

### Dosages des EEC

Les dosages *in vitro* du caractère répondeur érythroïde à l'Epo ont tous été réalisés dans le même laboratoire (Hôtel Dieu, Paris) avec une technique de culture de plasma-caillot, comme décrit précédemment (Casadevall N, Dupuy E, Molho-Sabatier P, Tobelem G, Varet B, Mayeux P. Autoantibodies against erythropoietin in a patient with pure red-cell aplasia. N. Engl. J. Med. 1996 ; 334 : 630 à 633).

### Analyse statistique

La corrélation des marqueurs a été réalisée en utilisant le coefficient de rangs Spearman (R).

### 2.2 RESULTATS

### Faisabilité et sensibilité des techniques de génotypage basées sur la PCR pour la détection de la mutation JAK2 V617F

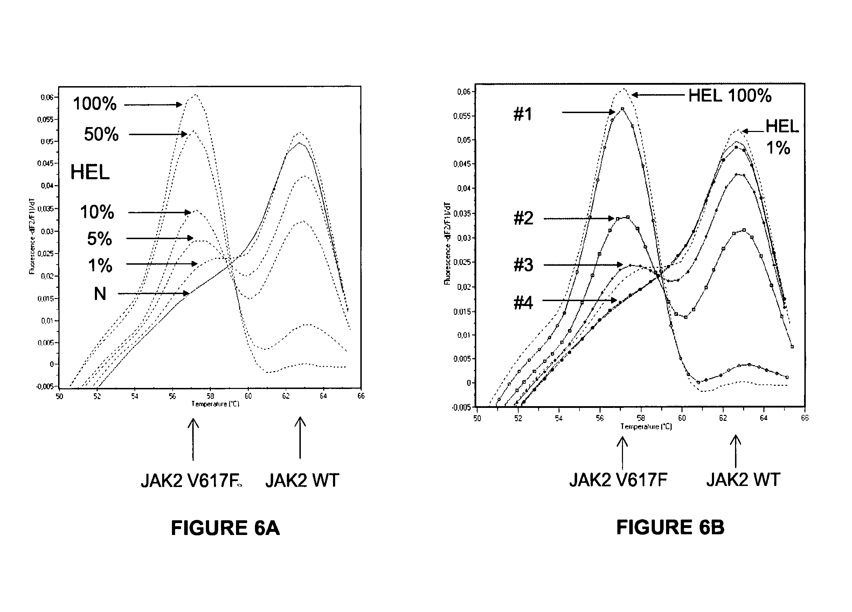

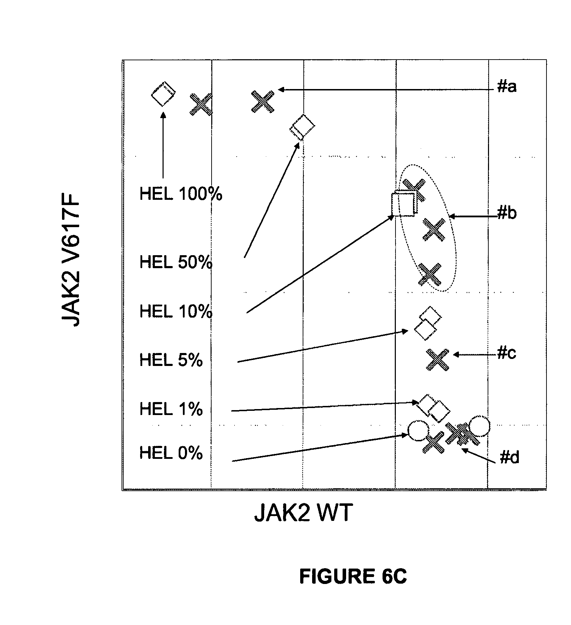

Pour évaluer l'efficacité du séquençage, des technologies LightCycler® et Taqman® pour détecter la mutation JAK2 V617F, nous avons cherché sa présence dans 119 échantillons provenant de patients avec une suspicion de MPD, en utilisant les 3 techniques en parallèle. La mutation JAK2 V617F a été efficacement détectée dans 83/119 échantillons, et 35 échantillons ne présentaient la mutation par aucune des 3 techniques. Dans un échantillon seulement, le séquençage a échoué à détecter la mutation révélée par les deux technologies LightCycler® et Taqman®, suggérant ainsi que ces dernières puissent être plus sensibles.
Pour évaluer la sensibilité de la technique, nous avons utilisé deux méthodes différentes : nous avons testé des dilutions en série de l'ADN de la lignée cellulaire HEL mutée de façon homozygote dans l'ADN de la lignée cellulaire TF-1 non mutée, et des dilutions en série de l'ADN génomique provenant d'un patient homozygote pour la mutation JAK2 V617F dans de l'ADN normal. Le séquençage a échoué à détecter l'allèle muté sous 5% d'ADN de lignée cellulaire HEL dilué dans de l'ADN de lignée cellulaire TF-1, et sous 10% de l'ADN du patient muté de façon homozygote dilué dans de l'ADN normal. La sensibilité des techniques LightCycler® et Taqman® était équivalente, légèrement supérieure au séquençage, atteignant 0,5 à 1% de l'ADN de la lignée cellulaire HEL dilué dans de l'ADN de la lignée cellulaire TF-1 (figure 6), et 2 à 4% de l'ADN d'un patient muté de façon homozygote dilué dans de l'ADN normal.

### Caractéristiques des patients avec une érythrocytose au moment du diagnostic

Les caractéristiques principales de 88 patients avec des taux d'hématocrites supérieurs à 51%, au moment du diagnostic, sont résumées dans le tableau 1.

**Tableau 1 : Caractéristiques des patients**

| | Critère WHO | | Critère PVSG | | Critères WHO et PVSG | |
|---|---|---|---|---|---|---|
| | PV n=61 | Erythrocytose idiopathique n= 11 | PV n=45 | Erythrocytose idiopathique n=21 | Erythrocytose secondaire n=5 | Ht>50%, mais pas d'AE n=3 |
| Rapport des sexes (mâle/femelle) | 38/23 | 11/0 | 28/17 | 18/3 | 4/1 | 3/0 |
| Age moyen (domaine) | 61 (23 à 92) | 57 (24 à 81) | 58(23 à 92) | 60 (53 à 81) | 65 (55 à 77) | 48,6 |
| Ht moyens (%) +/-σ | 59 +/- 4,6 | 54,6 +/- 1,44 | 59,2 +/- 4,5 | 57,8 +/- 4,2 | 55,8 +/- 3,1 | 53,3 +/- 0,8 |
| Hb moyenne (g/dL) +/- σ | 19,2 +/- 1,39 | 18,3 +/- 0,34 | 19,3 +/-1,41 | 19 +/- 1,0 | 18,9 +/- 0,8 | 18,6 +/- 0,5 |
| WBC moyens (x10⁹)+/-σ | 12,2 +/- 4,4 | 7,0 +/- 2,5 | 13,5 +/- 4,9 | 8,2 +/- 2,5 | 8,8 +/- 1,9 | 6,6 +/- 0,4 |
| Compte des plaquettes moyen (x10⁹)+/-σ | 463 +/- 148 | 212+/-38 | 503 +/- 149 | 245+/-60,4 | 212+/-29 | 175 +/-19 |
| Splénomégalie | 16/55 | 0/11 | 14/39 | 0/21 | 0/5 | 0/3 |
| Présence d'EEC | 59/60 | 1/11 | 43/44 | 11/21 | 0/5 | 0/3 |
| Niveau d'Epo bas | 39/47 | 2/8 | 27/33 | 10/17 | 0/3 | 1/1 |
| Niveau d'Epo normal | 8/47 | 6/8 | 6/33 | 7/17 | 3/3 | 0/1 |
| Anomalies cytogénétiques | 7/32 | 0/3 | 6/23 | 0/7 | nd | 0/1 |
| JAK2 V617F positive | 57/61 | 0/11 | 43/45 | 8/21 | 0/5 | 0/3 |

88 patients avec des taux d'hématocrites supérieurs à 51 % ont été diagnostiqués selon les critères PVSG et WHO, en quatre groups : maladie de Vasquez (PV), érythrocytose idiopathique, érythrocytose secondaire et « pas d'érythrocytose absolue » (pas d'AE) quand la mesure de la masse des globules rouges n'avait pas augmenté. 8 patients ne pouvaient avoir aucun diagnostic défini, puisque quelques données cliniques n'étaient pas disponibles. Ht : hématocrites ; Hb : hémoglobine ; WBC : globules blancs sanguins ; EEC : colonies érythroïdes endogènes ; Epo : érythropoïétine ; σ: écart type. Les patients pouvaient être séparés en 4 groupes principaux, respectivement selon les critères WHO (Pierre R. et al., éditeurs. World Health Organization Classification of Tumours ; Pathology and Genetics of tumours of hematopoietic and lymphoid tissues. Lyon : IARC Press ; 2001 : 32 à 34) et PVSG (Pearson TC, Messinezy M. The diagnostic criteria of polycythaemia rubra vera. Leuk Lymphoma 1996 : 22 Suppl 1 : 87 à 93) : 61 à 45 patients avec le diagnostic de PV, 5 avec une érythrocytose secondaire, 11 à 21 avec une érythrocytose idiopathique et 3 sans érythrocytose absolue (masse des globules rouges normale). Les données cliniques étaient incomplètes pour 7 patients, expliquant pourquoi le diagnostic de PV ne pouvait pas être affirmé soit avec les critères WHO soit avec ceux PVSG. En raison des différences entre les critères A1 des deux classifications, 6 patients qui n'avaient aucune mesure de masse de globules rouges, pouvaient être classés dans la classification WHO et pas dans celle PVSG. Un patient avait à la fois une hypoxie et une formation d'EEC, rendant par conséquent le diagnostic difficile. Une analyse cytogénétique a été réalisée chez 35 patients ; parmi 32 patients PV (selon le critère WHO), 7 avaient des anomalies cytogénétiques : 5 avec une trisomie 9, 1 avec 7q- et 1 avec du matériel supplémentaire sur le chromosome 18.

### La présence de JAK2 V617F correspond aux critères PVSG et WHO de PV

JAK2 V617F était présente dans 43/45 (96%) patients diagnostiqués en tant que PV selon les critères PVSG et dans 57/61 (93%) patients diagnostiqués avec les critères WHO (tableau 1). Néanmoins, 8/29 patients classés en tant que non PV selon la classification PVSG présentaient la mutation, alors qu'aucun des 19 patients non PV du WHO ; ces 8 patients étaient considérés IE avec les critères PVSG et PV avec ceux WHO. Aucun des patients diagnostiqués SE ni celui avec une masse de globules rouges normale (« pas d'AE ») n'avaient la mutation. La présence ou l'absence de JAK2 V617F correspond ainsi au diagnostic positif de PV dans 76/80 patients (95%, R = 0,879, p<0,0001) si on utilise les critères WHO et dans 64/74 patients (86,5%, R = 0,717, p<0,0001) avec les critères PVSG. En outre, puisqu'aucun des patients diagnostiqués en tant que non PV avec les critères WHO n'avait la mutation, la détection de JAK2 V617F a une valeur prédictive de 100% dans le contexte de l'érythrocytose absolue.
Quelques auteurs (Mossuz P. et al., Diagnostic value of serum erythropoietin level in patients with absolute erythrocytosis. Haematologica 2004 ; 89 : 1194 à 1198) considèrent la mesure du niveau d'Epo sérique comme un test de diagnostic en première intention pour les patients avec une érythrocytose absolue, avec une spécificité de 97% et une valeur prédictive de 97,8% pour le diagnostic de PV, si le niveau d'Epo est en dessous de la valeur inférieure du domaine normal. Dans notre étude, la corrélation entre le niveau d'Epo et le diagnostic de PV selon les critères WHO et PVSG a été observée respectivement dans 50/61 (82%, R = 0,488, p = 0,0002) et 39/56 (70%, R = 0,358, p = 0,0067) patients. Nous avons ensuite comparé le niveau d'Epo sérique en présence ou en l'absence de V617F JAK2 et on a trouvé que 52/68 patients (76%, R = 0,416, p = 0,0004) avaient une corrélation adéquate.
La présence de la mutation JAK2 V617F correspond à la capacité de former des EEC.
Trois équipes différentes ont montré que des lignées cellulaires dépendantes de l'Epo transfectées avec JAK2 V617F étaient indépendantes ainsi qu'hypersensibles à l'Epo pour leur croissance, mimant ainsi l'indépendance et l'hypersensibilité des progéniteurs érythroïdes décrits dans la PV. Par conséquent, nous avons émis l'hypothèse que les patients portant JAK2 V617F présentaient aussi une formation d'EEC. Parmi les 20 patients avec une érythrocytose qui n'avaient aucune formation d'EEC, un présentait la mutation, soulevant la question de la valeur prédictive positive de la détection de JAK2 V617F ; cependant, même si ce patient ne présentait aucune EEC, il remplissait de nombreux critères WHO et PVSG positifs, lui permettant d'être classé comme PV dans les deux classifications. Ce patient devrait par conséquent être considéré comme un « faux négatif aux EEC » plutôt que comme un « faux positif pour JAK2 ». Parmi les 67 patients qui avaient des EEC, 62 portaient la mutation JAK2 V617F, 5 patients n'étant pas mutés en utilisant des techniques sensibles pour la détection. Parmi ces 5 patients, 4/5 et 2/5 pouvaient être classés dans le groupe PV, respectivement selon les critères WHO et PVSG. Au total, les 87 patients analysés, la présence ou l'absence de la mutation JAK2 V617F correspondaient à la capacité ou à l'incapacité de former des EEC dans 81/87 patients (93,1%, R = 0,824, p<0,0001).

La présence de la mutation JAK2 V617F dans les cellules mononucléaires de la moelle osseuse (BMMC) correspond à sa présence dans les granulocytes périphériques.
Pour étudier si l'utilisation des granulocytes du sang périphérique pour détecter la mutation JAK2 V617F au moment du diagnostic pourrait éviter la réalisation du dosage sur les cellules de moelle osseuse, nous avons comparé les résultats obtenus par l'une ou l'autre des méthodes de séquençage, de LightCycler® et de TaqMan® chez 50 patients (incluant 35 PV, 8 SE et 8 autres suspicions de MPD) dont à la fois des échantillons de moelle osseuse et de sang périphérique étaient disponibles au moment du diagnostic. Dans tous les cas (34 mutés, 16 non mutés), la mutation a été détectée de façon identique.

### III- Conclusion

Nous proposons ainsi une nouvelle fiche de diagnostic de PV où la détection de JAK2 V617F dans les granulocytes avec des techniques sensibles est la première étape dans le diagnostic d'une érythrocytose, sauf dans le cas d'une érythrocytose secondaire évidente (figure 7). Cette attitude a plusieurs avantages : elle évite la réalisation d'une mesure de la masse des globules rouges isotopiques, qui n'est pas toujours disponible et dont le résultat est quelquefois sujet à controverse. Elle peut aussi éviter un aspirat de moelle osseuse et la réalisation des dosages d'EEC qui prennent du temps et ne sont pas bien standardisés. Elle diminue drastiquement le coût du diagnostic positif de PV, puisque seuls les patients avec des taux d'hématocrites supérieurs à 51% et négatives pour JAK2 V617F devraient avoir toutes les investigations qui sont réellement effectuées pour caractériser une érythrocytose. Même si la détection seule de JAK2 V617F dans le contexte de l'érythrocytose soutiendra le diagnostic de PV, la réalisation d'une biopsie de moelle osseuse peut encore être utile, puisqu'elle peut montrer des signes de myélofibrose ou la présence de cellules blastiques, affirmant par conséquent la transformation leucémique de la PV. Néanmoins, il nous semble qu'une biopsie de moelle osseuse devrait être réalisée dans le contexte d'une étude éventuelle, avec une analyse cytogénétique.
On a aussi détecté JAK2 V617F dans 30% des ET, 50% des IMF et quelques MPD rares non caractérisées, définissant par conséquent un nouveau groupe de MPD avec différentes présentations cliniques. Les raisons de ces différences sont encore inconnues et il est encore trop tôt pour regrouper ces maladies en une unique entité myéloproliférative avec une cause physiopathologique commune et différents phénotypes. Des études éventuelles cliniques précises caractériseraient plus précisément les signes communs entre PV, ET, IMF et d'autres MPD rares qui portent JAK2 V617F, spécialement en terme d'érythrocytose absolue, de niveau d'Epo, de myélofibrose et des anomalies cytogénétiques. On envisage ainsi d'utiliser la détection de JAK2 V617F en tant qu'outil initial dans le diagnostic de l'hyperleucocytose chronique, la thrombocytose et l'érythrocytose. La présence de JAK2 V617F ne permettra pas seulement une nouvelle définition d'un groupe de MPD, mais elle sera aussi certainement la base pour le développement de thérapies ciblées spécifiques.

### Exemple 3 : Les siRNA spécifiques de la mutation V617F JAK2 inhibent V617F JAK2 mais pas JAK2 WT.

Les siRNA 1, 3 et 4 correspondants aux SEQ ID No 25 à 27 inhibent la protéine mutée V617F JAK2 exprimée par la lignée HEL sans inhiber la protéine sauvage JAK2 exprimée par la lignée K562. Les résultats sont reportés aux figures 8, 9 et 10.

### REFERENCES

- Andersson, P., LeBlanc, K., Eriksson, B. A., and Samuelsson, J. (1997). No evidence for an altered mRNA expression or protein level of haematopoietic cell phosphatase in CD34+ bone marrow progenitor cells or mature peripheral blood cells in polycythaemia vera. Eur J Haematol 59, 310-7.
- Asimakopoulos, F. A., Hinshelwood, S., Gilbert, J. G., Delibrias, C. C., Gottgens, B., Fearon, D. T., and Green, A. R. (1997). The gene encoding hematopoietic cell phosphatase (SHP-1) is structurally and transcriptionally intact in polycythemia vera. Oncogene 14, 1215-22.
- Casadevall, N., Vainchenker, W., Lacombe, C., Vinci, J., Chapman, J., Breton-Gorius, J., and Varet, B. (1982). Erythroid progenitors in Polycythemia Vera. demonstration of their hypersensitivity to erythropoietin using serum-free cultures. Blood 59, 447-451.
- Hess, G., Rose, P., Gamm, H., Papadileris, S., Huber, C., and Seliger, B. (1994). Molecular analysis of the erythropoietin receptor system in patients with polycythaemia vera. Br J Haematol 88, 794-802.
- Kralovics, R., Guan, Y., and Prchal, J. T. (2002). Acquired uniparental disomy of chromosome 9p is a fréquent stem cell defect in polycythemia vera. Exp Hematol 30, 229-36.
- Le Couedic, J. P., Mitjavila, M. T., Villeval, J. L., Feger, F., Gobert, S., Mayeux, P., Casadevall, N., and Vainchenker, W. (1996). Missense mutation of the erythropoietin receptor is a rare event in human erythroid malignancies. Blood 87, 1502-11.
- Lindauer, K., Loerting, T., Liedl, K. R., and Kroemer, R. T. (2001). Prediction of the structure of human Janus kinase 2 (JAK2) comprising the two carboxy-terminal domains reveals a mechanism for autoregulation. Protein Eng 14, 27-37.
- Means, R. T., Krantz, S. B., Sawyer, S., and Gilbert, H. (1989). Erythropoietin receptors in polycythemia vera. J Clin Invest 84, 1340.
- Moliterno, A. R., Hankins, W. D., and Spivak, J. L. (1998). Impaired expression of the thrombopoietin receptor by platelets from patients with Polycythemia Vera. N Engl J Med 338, 572-580.
- Moliterno, A. R., and Spivak, J. L. (1999). Posttranslational processing of the thrombopoietin receptor is impaired in polycythemia vera. Blood 94, 2555-61.
- Pahl, H. L. (2000). Towards a molecular understanding of polycythemia rubra vera. Eur J Biochem 267, 3395-401.
- Pearson (2001). Evaluation of diagnostic criteria in polycythemia vera. Semin Hematol. 38, 21-4.
- Roder S, S. C., Meinhardt G, Pahl HL. (2001). STAT3 is constitutively active in some patients with Polycythemia rubra vera. Exp Hematol 29, 694-702.
- Silva, M., Richard, C., Benito, A., Sanz, C., Olalla, I., and Femandez-Luna, J. L. (1998). Expression of Bcl-x in erythroid precursors from patients with polycythemia vera. N Engl J Med 338, 564-71.
- Temerinac, S., Klippel, S., Strunck, E., Roder, S., Lubbert, M., Lange, W., Azemar, M., Meinhardt, G., Schaefer, H. E., and Pahl, H. L. (2000). Cloning of PRV-1, a novel member of the uPAR receptor superfamily, which is overexpressed in polycythemia rubra vera. Blood 95, 2569-76.
- Ugo, V., Marzac, C., Teyssandier, I., Larbret, F., Lécluse, Y., Debili, N., Vainchenker, W., and Casadevall, N. (2004). Multiple signaling pathways are involved in erythropoietin-independant differentiation of erythroid progenitors in Polycytyhemia Vera. Experimental Hematology 32, 179-187.

## Revendications

1. Protéine isolée JAK2 (Janus kinase 2), **caractérisée en ce qu'**elle comprend une mutation sur l'acide aminé 617, plus particulièrement la mutation V617F, ci-après dénommé « variant JAK2 V617F », dont la séquence est présentée à la SEQ ID No 1, ou des équivalents de cette protéine mutée en position 617 chez d'autres mammifères.

2. Séquence nucléotidique codant pour le variant JAK2 V617F selon la revendication 1, notamment la séquence SEQ ID No 2 possédant la mutation t/g en position 1849 à partir de l'ATG marquant le début de la transcription.

3. Vecteur de clonage et/ou d'expression viral, plasmidique ou sous forme d'ADN nu **caractérisé en ce qu'**il comprend la séquence selon la revendication 2 sous le contrôle d'un promoteur efficace dans les cellules de mammifères.

4. Cellule de mammifère, à l'exception de cellules souches humaines embryonnaires ou cellules souches germinales, exprimant le variant JAK2 V617F recombinant selon la revendication 1.

5. Animal transgénique non humain exprimant JAK2 V617F recombinant selon la revendication 1.

6. Animal transgénique non humain selon la revendication 5, **caractérisé en ce qu'**il s'agit d'une souris ou d'un rat ayant intégré dans son génome au moins la séquence codante pour JAK2 V617F par recombinaison homologue ou recombinaison dirigée.

7. Animal transgénique non humain selon la revendication 5 ou 6 **caractérisé en ce qu'**il est homozygote JAK2 V617F / JAK2 V617F ou hétérozygote JAK2 V617F / JAK2, lesdits animaux reproduisant la polyglobulie de Vaquez et/ou des syndromes myéloprolifératifs induits par JAK2 V617F.

8. Sondes ou amorces comprenant de 10 à 30 nucléotides consécutifs de la séquence SEQ ID No 3 ou 4 et comprenant le nucléotide muté t¹⁸⁴⁹.

9. Sondes ou amorces selon la revendication 8 sélectionnées parmi les séquences SEQ ID No 5 à 11 et 12.

10. Oligonucléotide d'au moins 10 nucléotides, notamment entre 15 et 25 nucléotides, capable de s'hybrider à l'ADN génomique de JAK2 de la séquence ID n°4 comprenant l'oligonucléotide muté t¹⁸⁴⁹, à l'ADNc ou l'ARNm.

11. Méthode *ex vivo* ou *in vitro* pour déterminer la présence ou l'absence du variant G 1849T du gène JAK2 dans un échantillon d'un patient atteint de PV ou susceptible de développer une PV ou tout autre syndrome myéloprolifératif, notamment de l'éthrocytose, les hyperleucocytoses, des thrombocytoses et myélofibroses, comprenant :
a) l'obtention d'un échantillon d'acide nucléique du patient,
b) la détection de la présence ou de l'absence du variant G1849T du gène JAK2 dans un échantillon d'acide nucléique, obtenu du patient
**caractérisée en ce que** la présence du variant G1849T est une indication de PV ou de tout autre syndrome myéloprolifératif.

12. Méthode selon la revendication 11, **caractérisée en ce qu'**elle comprend une étape d'hybridation avec au moins une sonde selon l'une des revendications 8 à 10.

13. Méthode selon la revendication 11 ou 12, **caractérisée en ce qu'**elle comprend une étape d'amplification par réaction PCR avec au moins une paire d'amorces selon l'une des revendications 8 à 10.

14. Méthode selon l'une des revendications 11 à 13, **caractérisée en ce qu'**elle est effectuée sur les ARNm d'un individu et comprenant une réaction RT-PCR.

15. Méthode selon l'une des revendications 11 à 13, **caractérisée en ce qu'**elle comprend une étape d'amplification au moyen desdites amorces, suivie par une étape d'hybridation avec au moins une sonde, de préférence deux sondes qui s'hybrident en condition de forte stringence aux séquences correspondant à la région de la mutation G 1849T et la détection du signal produit par les marqueurs desdites sondes, les sondes et amorces étant définies à la revendication 8 à 10.

16. Méthode *ex vivo* ou *in vitro* pour détecter la présence ou l'absence du variant JAK2 V617F dans un échantillon d'un patient atteint ou susceptible de développer la PV ou tout autre syndrome myéloprolifératif comprenant :
b) la détection de la présence ou de l'absence du variant JAK2 V617F dans un échantillon du patient,
**caractérisée en ce que** la présence dudit variant est une indication de la PV ou de tout autre syndrome myéloprolifératif.

17. Méthode selon la revendication 16 comprenant la mise en contact de l'échantillon avec un anticorps spécifique du variant V617F de la protéine JAK2, de préférence un anticorps capable de faire la distinction entre le variant V617F et la protéine JAK2 non mutée.

18. Méthode selon la revendication 17, **caractérisée en ce que** les anticorps sont des anticorps monoclonaux ou polyclonaux, simple chaîne ou double chaîne, des anticorps chimériques, humanisés ou des fragments de liaison à l'antigène F(ab')2 et F(v).

19. Méthode selon la revendication 18, **caractérisée en ce qu'**il s'agit d'un test ELISA.

20. Méthode selon l'une des revendications 11 à 19 **caractérisé en ce qu'**elle est pratiquée pour la sous-population de patients présentant un taux d'hématocrites supérieur à 51%.

21. Méthode selon l'une des revendications 11 à 19 **caractérisé en ce qu'**elle est pratiquée pour la sous-population de patients présentant un taux de plaquettes supérieur à 450 000.

22. Anticorps monoclonal reconnaissant spécifiquement le variant JAK2 V617F.

23. Hybridome produisant un anticorps selon la revendication 22.

24. Kit pour détecter le variant JAK2 V617F dans une tumeur comprenant une ou plusieurs amorces ou sondes telles que définies à la revendication 8 à 10 pour la détection spécifique de la présence ou de l'abscence de la mutation G1849T dans le gène JAK2.

25. Kit pour déterminer si un patient est atteint de la polyglobulie de Vaquez ou de tout autre syndrome myéloprolifératif impliquant le variant JAK2 V617F, notamment de l'éthrocytose, les hyperleucocytoses, des thrombocytoses et myélofibroses, comprenant une ou plusieurs sondes ou amorces telles que définies à la revendication 8 à 10 pour la détection spécifique de la présence ou de l'absence de la mutation G1849T dans le gène JAK2.

26. Kit selon la revendication 24 ou 25 comprenant en outre au moins un élément choisi parmi une polymérase thermorésistante pour l'amplification PCR, une ou plusieurs solutions pour l'amplification et/ou l'étape d'hybridation, ainsi que tout réactif permettant la détection du marqueur.

27. Kit pour déterminer si un patient est atteint de la polyglobulie de Vaquez ou de tout autre syndrome myéloprolifératif impliquant le variant JAK2 V617F comprenant un anticorps selon la revendication 22.

28. siRNA capable de diminuer de plus de 50% ou encore de plus de 95% l'expression du variant JAK2 V617F, **caractérisé en ce qu'**il possède de 19 à 25 nucléotides, de préférence 19 nucléotides de long, la séquence d'un premier brin étant identique et la séquence d'un deuxième brin étant complémentaire à la séquence SEQ ID No 3, SEQ ID No 4 ou encore la séquence SEQ ID No Il comprenant le nucléotide muté t ¹⁸⁴⁹.

29. Procédé in vitro permettant de déterminer l'inhibition spécifique de JAK2 V617F par un ou plusieurs composés comprenant les étapes consistant à :
a) mettre en contact un ou plusieurs composés avec la protéine JAK2 V617F selon la revendication 1, une fraction membranaire comprenant JAK2 V617F, ou encore une cellule exprimant JAK2 V617F selon la revendication 4 dans des conditions appropriées pour une fixation et/ou de l'inhibition et,
b) une détection de la fixation spécifique et/ou de l'inhibition de JAK2 V617F.

30. Procédé in vitro de criblage comprenant une succession de tests selon la revendication 29 de plusieurs molécules et une étape de sélection des molécules présentant une IC50 pour JAK2 V617F inférieure à 1 µM, de préférence 100 nM.

31. Procédé in vitro selon la revendication 30 comprenant en outre une sélection négative des molécules qui possèdent une IC50 pour JAK2 inférieure à 5 µM ou µM.

32. Procédé de criblage in vitro selon l'une des revendications 29 à 31 dans lequel on détermine par immunoprécipitation l'inhibition de la phosphorylation de JAK2 V617F.

33. Procédé de criblage in vitro selon l'une des revendications 29 à 32 sur des cellules primaires progénitrices CD34+ JAK2 V617F qui sont capables de se différencier sans érythropoïétine (Epo) ou sur des lignées cellulaires devenues facteur indépendantes par introduction du variant JAK2 V617F.

34. Procédé de criblage in vitro selon l'une des revendications 29 à 33 au moyen d'une cellule selon la revendication 4.

35. Procédé pour l'identification de candidats médicaments comprenant les étapes consistant à :
a) administrer des composés à un animal transgénique non humain exprimant JAK2 V617F tel que défini à l'une des revendications 5 à 7, ledit animal reproduisant la polyglobulie de vaquez et/ou étant atteint d'un syndrome myéloprolifératif associé à la présence de JAK2 V617F,
b) déterminer l'effet du composé et sélectionner les candidats médicaments pour lesquels on observe une diminution ou un blocage de la prolifération et de la différenciation spontanées des érythroblastes de polyglobulie de vaquez ou une diminution de la prolifération cellulaire associé à la présence de JAK2 V617F.

36. Utilisation des SiRNA selon la revendication 29 pour la fabrication d'un médicament.

37. Utilisation selon la revendication 36 pour la préparation d'un médicament destiné au traitement des hémopathies malignes, en particulier les syndromes myéloprolifératifs comprenant la Polyglobulie de Vaquez, la thrombocythémie essentielle, la splénomégalie myéloïde ou myélofibrose primitive.

38. Utilisation selon la revendication 36 pour la préparation d'un médicament destiné au traitement des syndromes myéloprolifératifs associées à la mutation JAK2 V617F, et des autres hémopathies malignes et des tumeurs solides tels que des carcinomes, des mélanomes et des neuroblastomes, qui expriment JAK2 V617F.

39. Composition comprenant un siRNA selon la revendication 28 et un véhicule pharmaceutiquement acceptable.

## Claims

1. Isolated JAK2 (Janus kinase 2) protein, **characterized in that** it comprises a mutation on amino acid 617, more particularly the V617F mutation, hereinafter referred to as "JAK2 V617F variant", the sequence of which is given in SEQ ID No. 1, or equivalents of this protein mutated at position 617 in other mammals.

2. Nucleotide sequence encoding the JAK2 V617F variant according to Claim 1, in particular the sequence SEQ ID No. 2 having the t/g mutation at position 1849 starting from the ATG marking the beginning of transcription.

3. Cloning and/or expression vector which is a viral or plasma vector or in the form of naked DNA, **characterized in that** it comprises the sequence according to Claim 2 under the control of a promoter which is effective in mammalian cells.

4. Mammalian cell, with the exception of human embryonic stem cells or germinal stem cells, expressing the recombinant JAK2 V617F variant according to Claim 1.

5. Non-human transgenic animal expressing recombinant JAK2 V617F according to Claim 1.

6. Non-human transgenic animal according to Claim 5, **characterized in that** it is a mouse or a rat having integrated into its genome at least the coding sequence for JAK2 V617F by homologous recombination or direct recombination.

7. Non-human transgenic animal according to Claim 5 or 6, **characterized in that** it is JAK2 V617F/JAK2 V617F homozygous or JAK2 V617F/JAK2 heterozygous, said animals reproducing polycythemia vera and/or myeloproliferative syndromes induced by JAK2 V617F.

8. Probes or primers comprising from 10 to 30 consecutive nucleotides of the sequence SEQ ID No. 3 or 4 and comprising the t¹⁸⁴⁹ mutated nucleotide.

9. Probes or primers according to Claim 8, selected from the sequences SEQ ID Nos. 5 to 11 and 12.

10. Oligonucleotide of at least 10 nucleotides, in particular between 15 and 25 nucleotides, capable of hybridizing to the JAK2 genomic DNA of sequence ID No. 4 comprising the t¹⁸⁴⁹ mutated oligonucleotide, to the cDNA or the mRNA.

11. *Ex vivo* or *in vitro* method for determining the presence or absence of the G1849T variant of the JAK2 gene in a sample from a patient suffering from PV or liable to develop a PV or any other myeloproliferative syndrome, in particular ethrocytosis, hyperleucocytosis, thrombocytosis and myelofibrosis, comprising:
a) obtaining a nucleic acid sample from the patient,
b) detecting the presence or absence of the G1849T variant of the JAK2 gene in a nucleic acid sample obtained from the patient,
**characterized in that** the presence of the G1849T variant is an indication of PV or of any other myeloproliferative syndrome.

12. Method according to Claim 11, **characterized in that** it comprises a step of hybridization with at least one probe according to one of Claims 8 to 10.

13. Method according to Claim 11 or 12, **characterized in that** it comprises a step of amplification by PCR reaction with at least one pair of primers according to one of Claims 8 to 10.

14. Method according to one of Claims 11 to 13, **characterized in that** it is carried out on the mRNA of an individual and comprising an RT-PCR reaction.

15. Method according to one of Claims 11 to 13, **characterized in that** it comprises an amplification step by means of said primers, followed by a step of hybridization with at least one probe, preferably two probes, which hybridize(s) under high stringency conditions to the sequences corresponding to the region of the G1849T mutation, and detection of the signal produced by the labels of said probes, the probes and primers being defined in Claims 8 to 10.

16. *ex vivo* or *in vitro* method for detecting the presence or absence of the JAK2 V617F variant in a sample from a patient suffering from or liable to develop PV or any other myeloproliferative syndrome, comprising:
b) detecting the presence or absence of the JAK2 V617F variant in a sample from the patient,
**characterized in that** the presence of said variant is an indication of PV or of any other myeloproliferative syndrome.

17. Method according to Claim 16, comprising bringing the sample into~contact with an antibody specific for the V617F variant of the JAK2 protein, preferably an antibody capable of distinguishing between the V617F variant and the non-mutated JAK2 protein.

18. Method according to Claim 17, **characterized in that** the antibodies are single-chain or double-chain, monoclonal or polyclonal antibodies, chimeric antibodies, humanized antibodies, or F(ab')2 and F(v) antigen-binding fragments.

19. Method according to Claim 18, **characterized in that** it is an ELISA assay.

20. Method according to one of Claims 11 to 19, **characterized in that** it is carried out for the subpopulation of patients having a hematocrit level of greater than 51%.

21. Method according to one of Claims 11 to 19, **characterized in that** it is carried out for the subpopulation of patients having a platelet count of greater than 450 000.

22. Monoclonal antibody which specifically recognizes the JAK2 V617F variant.

23. Hybridoma which produces an antibody according to Claim 22.

24. Kit for detecting the JAK2 V617F variant in a tumour, comprising one or more primers or probes as defined in Claims 8 to 10 for the specific detection of the presence or absence of the G1849T mutation in the JAK2 gene.

25. Kit for determining whether a patient is suffering from polycythemia vera or any other myeloproliferative syndrome involving the JAK2 V617F variant, in particular ethrocytosis, hyperleucocytosis, thrombocytosis and myelofibrosis, comprising one or more probes or primers as defined in Claims 8 to 10 for the specific detection of the presence or absence of the G1849T mutation in the JAK2 gene.

26. Kit according to Claim 24 or 25, also comprising at least one element chosen from a thermoresistant polymerase for PCR amplification, one or more solutions for the amplification and/or the hybridization step, and also any reagent for detecting the label.

27. Kit for determining whether a patient is suffering from polycythemia vera or any other myeloproliferative syndrome involving the JAK2 V617F variant, comprising an antibody according to Claim 22.

28. siRNA capable of decreasing by more than 50%, or else by more than 95%, the expression of the JAK2 V617F variant, **characterized in that** it has from 19 to 25 nucleotides, preferably 19 nucleotides in length, the sequence of a first strand being identical and the sequence of a second strand being complementary to the sequence SEQ ID No. 3 or SEQ ID No. 4 or else the sequence SEQ ID No. 11 comprising the t¹⁸⁴⁹ mutated nucleotide.

29. In *vitro* method for determining the specific inhibition of JAK2 V617F with one or more compounds, comprising the steps consisting in:
a) bringing one or more compounds into contact with the JAK2 V617F protein according to Claim 1, a membrane fraction comprising JAK2 V617F, or else a cell expressing JAK2 V617F according to Claim 4, under conditions suitable for binding and/or inhibition, and
b) detecting the specific binding and/or the inhibition of JAK2 V617F.

30. *In vitro* screening method comprising a succession of tests, according to Claim 29, of several molecules and a step of selection of the molecules exhibiting an IC50 for JAK2 V617F of less than 1 µM, preferably 100 nM.

31. *In vitro* method according to Claim 30, also comprising a negative selection of the molecules which have an IC50 for JAK2 of less than 5 µM or 1 µM.

32. *In vitro* screening method according to one of Claims 29 to 31, in which the inhibition of JAK2 V617F phosphorylation is determined by immuno-precipitation.

33. *In vitro* screening method according to one of Claims 29 to 32, on JAK2 V617F CD34+ primary progenitor cells which are capable of differentiating without erythropoietin (Epo) or on cell lines which have become factor-independent through the introduction of the JAK2 V617F variant.

34. *In vitro* screening method according to one of Claims 29 to 33, by means of a cell according to Claim 4.

35. Method for identifying medicament candidates, comprising the steps consisting in:
a) administering compounds to a non-human transgenic animal expressing JAK2 V617F as defined in one of Claims 5 to 7, said animal reproducing polycythemia vera and/or suffering from a myeloproliferative syndrome related to the presence of JAK2 V617F,
b) determining the effect of the compound and selecting the medicament candidates for which a decrease in or blocking of the spontaneous proliferation and differentiation of polycythemia vera erythroblasts or a decrease in cell proliferation related to the presence of JAK2 V617F is observed.

36. Use of the siRNAs according to Claim 29, for the manufacture of a medicament.

37. Use according to Claim 36, for the preparation of a medicament for use in the treatment of malignant haemopathies, in particular myeloproliferative syndromes including polycythemia vera, essential thrombocythemia, myeloid splenomegaly or primary myelofibrosis.

38. Use according to Claim 36, for the preparation of a medicament for use in the treatment of myeloproliferative syndromes related to the JAK2 V617F mutation, and of other malignant haemopathies and solid tumours such as carcinomas, melanomas and neuroblastomas, which express JAK2 V617F.

39. Composition comprising an siRNA according to Claim 28 and a pharmaceutically acceptable carrier.

## Patentansprüche

1. Isoliertes JAK2 (Janus-Kinase 2)-Protein, **dadurch gekennzeichnet, dass** es eine Mutation bezüglich der Aminosäure 617 umfasst, insbesondere die Mutation V617F, welche nachfolgend als "Variante JAK2 V617F" bezeichnet wird, deren Sequenz in der SEQ 1D No. 1 angegeben ist, oder Äquivalente von diesem an Position 617 mutierten Protein in anderen Säugetieren.

2. Nukleotidsequenz, welche die Variante JAK2 V617F nach Anspruch 1 kodiert, insbesondere die Sequenz SEQ ID No. 2, welche die Mutation t/g an Position 1849 ausgehend von dem ATG, welches den Beginn der Transkription bezeichnet, aufweist.

3. Virater, plasmidischer oder in Form von nackter DNA vorliegender Klonierungs- und/oder Expressionsvektor, **dadurch gekennzeichnet, dass** er die Sequenz nach Anspruch 2 unter der Kontrolle eines in den Säugetierzellen wirksamen Promotors umfasst.

4. Säugetierzelle mit Ausnahme von humanen embryonalen Stammzellen oder Keimstammzellen, welche die rekombinante Variante JAK2 V617F nach Anspruch 1 exprimiert.

5. Transgenes, nicht-humanes Tier, welches rekombinantes JAK2 V617F nach Anspruch 1 exprimiert.

6. Transgenes, nicht-humanes Tier nach Anspruch 5, **dadurch gekennzeichnet, dass** es sich um eine Maus oder eine Ratte handelt, in deren Genom wenigstens die JAK2 V617F kodierende Sequenz durch homologe Rekombination oder zielgerichtete Rekombination integriert worden ist.

7. Transgenes, nicht-humanes Tier nach Anspruch 5 oder 6, **dadurch gekennzeichnet, dass** es JAK2 V617F/JAK2 V617F-homozygot oder JAK2 V617/JAK2-heterozygot ist, wobei die Tiere die Vaquez-Polyzythämie und/oder myeloproliferative Syndrome, die durch JAK2 V617F induziert werden, reproduzieren.

8. Sonden oder Primer, welche 10 bis 30 aufeinanderfolgende Nukleotide der Sequenz SEQ ID No. 3 oder 4 umfassen und das mutierte Nukleotid t¹⁸⁴⁹ umfassen.

9. Sonden oder Primer nach Anspruch 8, die unter den Sequenzen SEQ ID No. 5 bis 11 und 12 ausgewählt werden.

10. Oligonukleotid von wenigstens 10 Nukleotiden, insbesondere zwischen 15 und 25 Nukleotiden, welches in der Lage ist, mit der genomischen DNA von JAK2 mit der Sequenz ID No. 4, umfassend das bezüglich t¹⁸⁴⁹ mutierte Oligonukleotid, mit der cDNA oder der mRNA zu hybridisieren.

11. *Ex vivo-* oder *in vitro*-Verfahren zum Bestimmen des Vorhandenseins oder des Fehlens der Variante G1849T des Gens JAK2 in einer Probe von einem Patienten, der an PV leidet oder eine PV oder ein jegliches anderes myeloproliferatives Syndrom, insbesondere Erythrozytose, Hyperleukozytosen, Thrombozytosen und Myelofibrosen, entwickeln könnte, umfassend:
a) die Gewinnung einer Nukleinsäureproben des Patienten;
b) den Nachweis des Vorhandenseins oder des Fehlens der Variante G1849T des Gens JAK2 in einer Nukleinsäureprobe, die von dem Patienten erhalten worden ist,
**dadurch gekennzeichnet, dass** das Vorhandensein der Variante G1849T ein Hinweis auf PV oder eines jeglichen anderen myeloproliferativen Syndroms ist.

12. Verfahren nach Anspruch 11, **dadurch gekennzeichnet, dass** es einen Hybridisierungsschritt mit wenigstens einer Sonde nach einem der Ansprüche 8 bis 10 umfasst.

13. Verfahren nach Anspruch 11 oder 12, **dadurch gekennzeichnet, dass** es einen Amplifizierungsschrftl durch PCR-Reaktion mit wenigstens einem Paar von Primern nach einem der Ansprüche 8 bis 10 umfasst.

14. Verfahren nach einem der Ansprüche 11 bis 13, **dadurch gekennzeichnet, dass** es an den mRNAs von einem Individuum ausgeführt wird und eine RT-PCR-Reaktion umfasst.

15. Verfahren nach einem der Ansprüche 11 bis 13, **dadurch gekennzeichnet, dass** es einen Amplifizierungsschritt mittels der Primer umfasst, gefolgt von einem Schritt einer Hybridisierung mit wenigstens einer Sonde, vorzugsweise zwei Sonden, die unter hoher Stringenz mit den der Region der Mutation G1849T entsprechenden Sequenzen hybridisieren, und dem Nachweis des durch die Marker der Sonden erzeugten Signals, wobei die Sonden und Primer in Anspruch 8 bis 10 definiert sind.

16. *Ex vivo-* oder *in vitro*-Verfahren zum Nachweisen des Vorhandenseins oder des Fehlens der Variante JAK2 V617F in einer Probe eines Patienten, der an PV leidet oder PV oder ein jegliches anderes myeloproliferatives Syndrom entwickeln könnte, umfassend:
b) den Nachweis des Vorhandenseins oder des Fehlens der Variante JAK2 V617F in einer **Probe des Patienten,**
**dadurch gekennzeichnet, dass** das Vorhandensein der Variante ein Hinweis auf PV oder ein jegliches anderes myeloproliferatives Syndrom ist.

17. Verfahren nach Anspruch 16, welches das Inkontaktbringen der Probe mit einem für die Variante V617F des Proteins JAK2 spezifischen Antikörper, vorzugsweise einem Antikörper, welcher in der Lage ist, zwischen der Variante V617F und dem nicht mutierten Protein JAK2 zu unterscheiden, umfasst.

18. Verfahren nach Anspruch 17, **dadurch gekennzeichnet, dass** die Antikörper monoklonale oder polyklonale, einzelkettige oder doppelkettige Antikörper, chimäre, humanisierte Antikörper oder Antigenbindungsfragmente F(ab')2 und F(v) sind.

19. Verfahren nach Anspruch 18, **dadurch gekennzeichnet, dass** es sich um einen ELISA-Test handelt.

20. Verfahren nach einem der Ansprüche 1 bis 19, **dadurch gekennzeichnet, dass** es an der Unterpopulation von Patienten, die einen Hämatokrit-Prozentsatz über 51% aufweisen, ausgeführt wird.

21. Verfahren nach einem der Ansprüche 1 bis 19, **dadurch gekennzeichnet, dass** es an der Unterpopulation von Patienten, die eine Thrombozytenzahl über 450000 aufweisen, ausgeführt wird.

22. Monoklonaler Antikörper, der spezifisch die Variante JAK2 V617F erkennt.

23. Hybridom, welches einen Antikörper nach Anspruch 22 produziert.

24. Kit zum Nachweisen der Variante JAK2 V617F in einem Tumor, umfassend einen oder mehrere Primer oder Sonden, wie in Anspruch 8 bis 10 definiert, für den spezifischen Nachweis des Vorhandenseins oder des Fehlens der Mutation G1849T in dem JAK2-Gen.

25. Kit zum Bestimmen, ob ein Patient unter der Vaquez-Polyzythämie oder einem jeglichen anderen myeloproliferativen Syndrom, an welchem die Variante JAK2 V617F beteiligt ist, insbesondere unter Erythrozytose, Hyperleukozytosen, Thrombozytosen und Myelofibrosen, leidet, umfassend eine oder mehrere Sonden oder Primer, wie in Anspruch 8 bis 10 definiert, für den spezifischen Nachweis des Vorhandenseins oder des Fehlens der Mutation G1849T in dem JAK2-Gen.

26. Kit nach Anspruch 24 oder 25, umfassend außerdem wenigstens ein Element, welches ausgewählt wird unter einer thermoresistenten Polymerase für die PCR-Amplifizierung, einer oder mehreren Lösungen für die Amplifizierung und/oder den Hybridisierungsschritt wie auch einem jeglichen Reagens, welches den Nachweis des Markers erlaubt.

27. Kit zum Bestimmen, ob ein Patient unter der Vaquez-Polyzythämie oder einem jeglichen anderen myeloproliferativen Syndrom, an welchem die Variante JAK2 V617F beteiligt ist, leidet, umfassend einen Antikörper nach Anspruch 22.

28. siRNA, welche in der Lage ist, die Expression der Variante JAK2 V617F um mehr als 50% oder ferner um mehr als 95% zu verringern, **dadurch gekennzeichnet, dass** sie 19 bis 25 Nukleotide aufweist, vorzugsweise mit einer Länge von 19 Nukleotiden, wobei die Sequenz eines ersten Strangs identisch ist mit und die Sequenz eines zweiten Strangs komplementär ist zu der Sequenz SEQ ID No. 3, SEQ ID No. 4 oder ferner der Sequenz SEQ ID No. 11, umfassend das mutierte Nukleotid t¹⁸⁴⁹.

29. *In vitro*-Verfahren, welches erlaubt, die spezifische Inhibition von JAK2 V617F durch eine oder mehrere Verbindungen zu bestimmen, welches die Schritte umfasst, welche daraus bestehen:
a) eine oder mehrere Verbindungen mit dem Protein JAK2 V617F nach Anspruch 1, einer JAK2 V617F umfassenden Membranfraktion oder ferner einer JAK2 V617F exprimierenden Zelle nach Anspruch 4 unter Bedingungen, die für eine Bindung und/oder die Inhibition geeignet sind, in Kontakt zu bringen und
b) einem Nachweis der spezifischen Bindung und/oder der Inhibition von JAK2 V617F.

30. *In vitro*-Screeningverfahren, umfassend eine Aufeinanderfolge von Tests nach Anspruch 29 von mehreren Molekülen und einen Schritt einer Auswahl der Moleküle, die eine IC50 für JAK2 V617F unter 1 µM. vorzugsweise 100 nM aufweisen.

31. *In vitro*-Verfahren nach Anspruch 30, umfassend außerdem eine negative Selektion der Moleküle, die eine IC50 für JAK2 unter 5 µM oder 1 µM aufweisen.

32. *In vitro*-Screeningverfahren nach einem der Ansprüche 29 bis 31, in welchem man durch Immunpräzipitation die Inhibition der Phosphorylierung von JAK2 V617F bestimmt.

33. *In vitro*-Screeningverfahren nach einem der Ansprüche 29 bis 32 an primären CD34+ JAK2 V617F-Vorläuferzellen, die in der Lage sind, ohne Erythropoietin (Epo) zu differenzieren, oder an Zelllinien, die durch Einführung der Variante JAK2 V617F Faktor-unabhängig geworden sind.

34. *In vitro*-Screeningverfahren nach einem der Ansprüche 29 bis 33 mittels einer Zelle nach Anspruch 4.

35. Verfahren zur Identifizierung von Arzneimittelkandidaten, welches die Schritte umfasst, die daraus bestehen:
a) Verbindungen an ein transgenes, nicht-humanes Tier, welches JAK2 V617F exprimiert, wie in einem der Ansprüche 5 bis 7 definiert, zu verabreichen, wobei das Tier die Vaquez-Polyzythämie reproduziert und/oder an einem myeloproliferativen Syndrom, das mit dem Vorhandensein von JAK2 V617F in Zusammenhang steht, leidet,
b) die Wirkung der Verbindung zu bestimmen und die Arzneimittelkandidaten auszuwählen, für welche man eine Verringerung oder eine Blockierung der Proliferation und der spontanen Differenzierung der Erythroblasten von Vaquez-Polyzythämie oder eine Verringerung der Zellproliferation, die mit dem Vorhandensein von JAK2 V617F in Zusammenhang steht, beobachtet.

36. Verwendung der SiRNAs nach Anspruch 29 für die Herstellung eines Arzneimittels.

37. Verwendung nach Anspruch 36 für die Herstellung eines Arzneimittels, welches für die Behandlung von malignen Hämopathien, insbesondere der myeloproliferativen Syndrome, umfassend die Vaquez-Polyzythämie, die essentielle Thrombozythämie, die myeloische Splenomegalie oder die primitive Myelofibrose, bestimmt ist.

38. Verwendung nach Anspruch 36 für die Herstellung eines Arzneimittels, welches für die Behandlung der mit der Mutation JAK2 V617F in Zusammenhang stehenden myeloproliferativen Syndrome und von anderen malignen Hämopathien und von soliden Tumoren, wie Karzinomen, Melanomen und Neuroblastomen, die JAK2 V617F exprimieren, bestimmt ist.

39. Zusammensetzung, welche eine siRNA nach Anspruch 28 und einen pharmazeutisch annehmbaren Träger umfasst.


### LISTE DE SEQUENCES

<110> Assistance Publique - Hôpitaux de Paris (AH-HP)
   Institut National de la Santé et de la Recherche Médicale
   (INSERM)
   Institut Gustave Roussy (IGR)
   Université de Versailles - Saint-Quentin-en-Yvelines
   Université Paris-Sud
<120> Identification d'une mutation de JAK2 impliquée dans le Polyglobulie de Vaquez
<130> D 22 707 / 241 699
<140> FR04-11480
   <141> 2004-10-27
<160> 31
<170> PatentIn version 3.3
<210> 1
   <211> 1132
   <212> PRT
   <213> homo sapiens
<220>
   <223> variant JAK2 V617F.
<400> 1
<210> 2
   <211> 5097
   <212> ADN
   <213> homo sapiens
<220>
   <223> mutation G1849T dans le gène jak2.
<400> 2
   ctgcaggaag gagagaggaa gaggagcaga agggggcagc agcggacgcc gctaacggcc 60
   tccctcggcg ctgacaggct gggccggcgc ccggctcgct tgggtgttcg cgtcgccact 120
   tcggcttctc ggccggtcgg gcccctcggc ccgggcttgc ggcgcgcgtc ggggctgagg 180
   gctgctgcgg cgcagggaga ggcctggtcc tcgctgccga gggatgtgag tgggagctga 240
   gcccacactg gagggccccc gagggcccag cctggaggtc gttcagagcc gtgcccgccc 300
   cggggcttcg cagaccttga cccgccgggt aggagccgcc cctgcgggct cgagggcgcg 360
   ctctggtcgc ccgatctgtg tagccggttt cagaagcagg caacaggaac aagatgtgaa 420
   ctgtttctct tctgcagaaa aagaggctct tcctcctcct cccgcgacgg caaatgttct 480
   gaaaaagact ctgcatggga atggcctgcc ttacgatgac agaaatggag ggaacatcca 540
   cctcttctat atatcagaat ggtgatattt ctggaaatgc caattctatg aagcaaatag 600
   atccagttct tcaggtgtat ctttaccatt cccttgggaa atctgaggca gattatctga 660
   cctttccatc tggggagtat gttgcagaag aaatctgtat tgctgcttct aaagcttgtg 720
   gtatcacacc tgtgtatcat aatatgtttg ctttaatgag tgaaacagaa aggatctggt 780
   atccacccaa ccatgtcttc catatagatg agtcaaccag gcataatgta ctctacagaa 840
   taagatttta ctttcctcgt tggtattgca gtggcagcaa cagagcctat cggcatggaa 900
   tatctcgagg tgctgaagct cctcttcttg atgactttgt catgtcttac ctctttgctc 960
   agtggcggca tgattttgtg cacggatgga taaaagtacc tgtgactcat gaaacacagg 1020
   aagaatgtct tgggatggca gtgttagata tgatgagaat agccaaagaa aacgatcaaa 1080
   ccccactggc catctataac tctatcagct acaagacatt cttaccaaaa tgtattcgag 1140
   caaagatcca agactatcat attttgacaa ggaagcgaat aaggtacaga tttcgcagat 1200
   ttattcagca attcagccaa tgcaaagcca ctgccagaaa cttgaaactt aagtatctta 1260
   taaatctgga aactctgcag tctgccttct acacagagaa atttgaagta aaagaacctg 1320
   gaagtggtcc ttcaggtgag gagatttttg caaccattat aataactgga aacggtggaa 1380
   ttcagtggtc aagagggaaa cataaagaaa gtgagacact gacagaacag gatttacagt 1440
   tatattgcga ttttcctaat attattgatg tcagtattaa gcaagcaaac caagagggtt 1500
   caaatgaaag ccgagttgta actatccata agcaagatgg taaaaatctg gaaattgaac 1560
   ttagctcatt aagggaagct ttgtctttcg tgtcattaat tgatggatat tatagattaa 1620
   ctgcagatgc acatcattac ctctgtaaag aagtagcacc tccagccgtg cttgaaaata 1680
   tacaaagcaa ctgtcatggc ccaatttcga tggattttgc cattagtaaa ctgaagaaag 1740
   caggtaatca gactggactg tatgtacttc gatgcagtcc taaggacttt aataaatatt 1800
   ttttgacttt tgctgtcgag cgagaaaatg tcattgaata taaacactgt ttgattacaa 1860
   aaaatgagaa tgaagagtac aacctcagtg ggacaaagaa gaacttcagc agtcttaaag 1920
   atcttttgaa ttgttaccag atggaaactg ttcgctcaga caatataatt ttccagttta 1980
   ctaaatgctg tcccccaaag ccaaaagata aatcaaacct tctagtcttc agaacgaatg 2040
   gtgtttctga tgtaccaacc tcaccaacat tacagaggcc tactcatatg aaccaaatgg 2100
   tgtttcacaa aatcagaaat gaagatttga tatttaatga aagccttggc caaggcactt 2160
   ttacaaagat ttttaaaggc gtacgaagag aagtaggaga ctacggtcaa ctgcatgaaa 2220
   cagaagttct tttaaaagtt ctggataaag cacacagaaa ctattcagag tctttctttg 2280
   aagcagcaag tatgatgagc aagctttctc acaagcattt ggttttaaat tatggagtat 2340
   gtttctgtgg agacgagaat attctggttc aggagtttgt aaaatttgga tcactagata 2400
   catatctgaa aaagaataaa aattgtataa atatattatg gaaacttgaa gttgctaaac 2460
   agttggcatg ggccatgcat tttctagaag aaaacaccct tattcatggg aatgtatgtg 2520
   ccaaaaatat tctgcttatc agagaagaag acaggaagac aggaaatcct cctttcatca 2580
   aacttagtga tcctggcatt agtattacag ttttgccaaa ggacattctt caggagagaa 2640
   taccatgggt accacctgaa tgcattgaaa atcctaaaaa tttaaatttg gcaacagaca 2700
   aatggagttt tggtaccact ttgtgggaaa tctgcagtgg aggagataaa cctctaagtg 2760
   ctctggattc tcaaagaaag ctacaatttt atgaagatag gcatcagctt cctgcaccaa 2820
   agtgggcaga attagcaaac cttataaata attgtatgga ttatgaacca gatttcaggc 2880
   cttctttcag agccatcata cgagatctta acagtttgtt tactccagat tatgaactat 2940
   taacagaaaa tgacatgtta ccaaatatga ggataggtgc cctagggttt tctggtgcct 3000
   ttgaagaccg ggatcctaca cagtttgaag agagacattt gaaatttcta cagcaacttg 3060
   gcaagggtaa ttttgggagt gtggagatgt gccggtatga ccctctacag gacaacactg 3120
   gggaggtggt cgctgtaaaa aagcttcagc atagtactga agagcaccta agagactttg 3180
   aaagggaaat tgaaatcctg aaatccctac agcatgacaa cattgtaaag tacaagggag 3240
   tgtgctacag tgctggtcgg cgtaatctaa aattaattat ggaatattta ccatatggaa 3300
   gtttacgaga ctatcttcaa aaacataaag aacggataga tcacataaaa cttctgcagt 3360
   acacatctca gatatgcaag ggtatggagt atcttggtac aaaaaggtat atccacaggg 3420
   atctggcaac gagaaatata ttggtggaga acgagaacag agttaaaatt ggagattttg 3480
   ggttaaccaa agtcttgcca caagacaaag aatactataa agtaaaagaa cctggtgaaa 3540
   gtcccatatt ctggtatgct ccagaatcac tgacagagag caagttttct gtggcctcag 3600
   atgtttggag ctttggagtg gttctgtatg aacttttcac atacattgag aagagtaaaa 3660
   gtccaccagc ggaatttatg cgtatgattg gcaatgacaa acaaggacag atgatcgtgt 3720
   tccatttgat agaacttttg aagaataatg gaagattacc aagaccagat ggatgcccag 3780
   atgagatcta tatgatcatg acagaatgct ggaacaataa tgtaaatcaa cgcccctcct 3840
   ttagggatct agctcttcga gtggatcaaa taagggataa catggctgga tgaaagaaat 3900
   gaccttcatt ctgagaccaa agtagattta cagaacaaag ttttatattt cacattgctg 3960
   tggactatta ttacatatat cattattata taaatcatga tgctagccag caaagatgtg 4020
   aaaatatctg ctcaaaactt tcaaagttta gtaagttttt cttcatgagg ccaccagtaa 4080
   aagacattaa tgagaattcc ttagcaagga ttttgtaaga agtttcttaa acattgtctg 4140
   ttaacatcac tcttgtctgg caaaagaaaa aaaatagact ttttcaactc agctttttga 4200
   gacctgaaaa aattattatg taaattttgc aatgttaaag atgcacagaa tatgtatgta 4260
   tagtttttac cacagtggat gtataatacc ttggcatctt gtgtgatgtt ttacacacat 4320
   gagggctggt gttcattaat actgttttct aatttttcca tagttaatct ataattaatt 4380
   acttcactat acaaacaaat taagatgttc agataattga ataagtacct ttgtgtcctt 4440
   gttcatttat atcgctggcc agcattataa gcaggtgtat acttttagct tgtagttcca 4500
   tgtactgtaa atatttttca cataaaggga acaaatgtct agttttattt gtataggaaa 4560
   tttccctgac cctaaataat acattttgaa atgaaacaag cttacaaaga tataatctat 4620
   tttattatgg tttcccttgt atctatttgt ggtgaatgtg ttttttaaat ggaactatct 4680
   ccaaattttt ctaagactac tatgaacagt tttcttttaa aattttgaga ttaagaatgc 4740
   caggaatatt gtcatccttt gagctgctga ctgccaataa cattcttcga tctctgggat 4800
   ttatgctcat gaactaaatt taagcttaag ccataaaata gattagattg ttttttaaaa 4860
   atggatagct cattaagaag tgcagcaggt taagaatttt ttcctaaaga ctgtatattt 4920
   gaggggtttc agaattttgc attgcagtca tagaagagat ttatttcctt tttagagggg 4980
   aaatgaggta aataagtaaa aaagtatgct tgttaatttt attcaagaat gccagtagaa 5040
   aattcataac gtgtatcttt aagaaaaatg agcatacatc ttaaatcttt tcaatta 5097
<210> 3
   <211> 554
   <212> ADN
   <213> homo sapiens
<220>
   <223> fragment de la SEQ ID No 2 avec la mutation G1849T.
<400> 3
   ctcatatgaa ccaaatggtg tttcacaaaa tcagaaatga agatttgata tttaatgaaa     60
   gccttggcca aggcactttt acaaagattt ttaaaggcgt acgaagagaa gtaggagact    120
   acggtcaact gcatgaaaca gaagttcttt taaaagttct ggataaagca cacagaaact    180
   attcagagtc tttctttgaa gcagcaagta tgatgagcaa gctttctcac aagcatttgg    240
   ttttaaatta tggagtatgt ttctgtggag acgagaatat tctggttcag gagtttgtaa    300
   aatttggatc actagataca tatctgaaaa agaataaaaa ttgtataaat atattatgga    360
   aacttgaagt tgctaaacag ttggcatggg ccatgcattt tctagaagaa aacaccctta    420
   ttcatgggaa tgtatgtgcc aaaaatattc tgcttatcag agaagaagac aggaagacag    480
   gaaatcctcc tttcatcaaa cttagtgatc ctggcattag tattacagtt ttgccaaagg    540
   acattcttca ggag                                                      554
<210> 4
   <211> 94
   <212> ADN
   <213> homo sapiens
<220>
   <223> fragment de la SEQ ID No 3 avec la mutation G1849T.
<400> 4
   gatgagcaag ctttctcaca agcatttggt tttaaattat ggagtatgtt tctgtggaga     60
   cgagaatatt ctggttcagg agtttgtaaa attt                                 94
<210> 5
   <211> 20
   <212> ADN
   <213> Séquence artificielle.
<220>
   <223> AMORCE DE PCR (54804-54823).
<400> 5
   gggtttcctc agaacgttga 20
<210> 6
   <211> 21
   <212> ADN
   <213> Séquence artificielle.
<220>
   <223> AMORCE DE PCR (55240-55260).
<400> 6
   ttgctttcct ttttcacaag a 21
<210> 7
   <211> 20
   <212> ADN
   <213> Séquence artificielle.
<220>
   <223> AMORCE DE SEQUENÇAGE (54813-54832).
<400> 7
   cagaacgttg atggcagttg 20
<210> 8
   <211> 27
   <212> ADN
   <213> Séquence artificielle.
<220>
   <223> AMORCE DE SEQUENÇAGE (55207-55233).
<400> 8
   tgaatagtcc tacagtgttt tcagttt 27
<210> 9
   <211> 21
   <212> ADN
   <213> Séquence artificielle.
<220>
   <223> AMORCE DE PCR ET SEQUENÇAGE (1386-1407).
<400> 9
   caacctcagt gggacaaaga a 21
<210> 10
   <211> 23
   <212> ADN
   <213> Séquence artificielle.
<220>
   <223> AMORCE DE PCR ET SEQUENÇAGE (2019-2041).
<400> 10
   gcagaatatt tttggcacat aca 23
<210> 11
   <211> 42
   <212> ADN
   <213> Séquence artificielle.
<220>
   <223> SONDES SNPS ET DETECTION DE MUTATION ET siRNA (1829-1870).
<400> 11
   ttttaaatta tggagtatgt gtctgtggag acgagaatat tc 42
<210> 12
   <211> 23
   <212> ADN
   <213> homo sapiens
<220>
   <223> Séquence comprenant la mutation G1849T.
<400> 12
   tatggagtat gtttctgtgg aga 23
<210> 13
   <211> 21
   <212> ARN
   <213> homo sapiens
<220>
   <221> misc_feature
   <222> (20)..(21)
   <223> n est T
<220>
   <223> siRNA sens
<400> 13
   uggaguaugu uucuguggan n 21
<210> 14
   <211> 21
   <212> ARN
   <213> homo sapiens
<220>
   <221> misc_feature
   <222> (20) ..(21)
   <223> n est T
<220>
   <223> siRNA antisens.
<400> 14
   uccacagaaa cauacuccan n 21
<210> 15
   <211> 21
   <212> ADN
   <213> Séquence artificielle
<220>
   <223> Oligo "S" (sens)
<400> 15
   ggcagagaga attttctgaa c 21
<210> 16
   <211> 21
   <212> ADN
   <213> Séquence artificielle
<220>
   <223> oligo "R" (antisens)
<400> 16
   gctttccttt ttcacaagat a 21
<210> 17
   <211> 25
   <212> ADN
   <213> Séquence artificielle
<220>
   <223> "Sensor wt"
<400> 17
   gtctccacag acacatactc cataa 25
<210> 18
   <211> 25
   <212> ADN
   <213> Séquence artificielle
<220>
   <223> amorce JAK2
<400> 18
   aaaaccaaat gcttgtgaga aagct 25
<210> 19
   <211> 23
   <212> ADN
   <213> Séquence artificielle
<220>
   <223> cJAK2F
<400> 19
   gcacacagaa actattcaga gtc 23
<210> 20
   <211> 19
   <212> ADN
   <213> Séquence artificielle
<220>
   <223> cJAK2S
<400> 20
   agcagcaagt atgatgagc 19
<210> 21
   <211> 24
   <212> ADN
   <213> Séquence artificielle
<220>
   <223> cJAK2A
<400> 21
   ctagtgatcc aaattttaca aact 24
<210> 22
   <211> 21
   <212> ADN
   <213> Séquence artificielle
<220>
   <223> cJAK2R
<400> 22
   gtttagcaac ttcaagtttc c 21
<210> 23
   <211> 25
   <212> ADN
   <213> Séquence artificielle
<220>
   <223> "Sensor wt"
<400> 23
   gtctccacag acacatactc cataa 25
<210> 24
   <211> 25
   <212> ADN
   <213> Séquence artificielle
<220>
   <223> Amorce JAK2
<400> 24
   aaaaccaaat gcttgtgaga aagct 25
<210> 25
   <211> 25
   <212> ADN
   <213> Séquence artificielle
<220>
   <223> Séquence de l'amorce sens
<400> 25
   aagctttctc acaagcattt ggttt 25
<210> 26
   <211> 26
   <212> ADN
   <213> Séquence artificielle
<220>
   <223> Séquence de l'amorce antisens
<400> 26
   agaaaggcat tagaaagcct gtagtt 26
<210> 27
   <211> 17
   <212> ADN
   <213> Séquence artificielle
<220>
   <223> Séquence du "Reporter 1" (VIC)
<400> 27
   tctccacaga cacatac 17
<210> 28
   <211> 15
   <212> ADN
   <213> Séquence artificielle
<220>
   <223> Séquence du "Reporter 2" (FAM)
<400> 28
   tccacagaaa catac 15
<210> 29
   <211> 19
   <212> ARN
   <213> Séquence artificielle
<220>
   <223> siRNA
<400> 29
   uggaguaugu uucugugga 19
<210> 30
   <211> 19
   <212> ARN
   <213> Séquence artificielle
<220>
   <223> siRNA
<400> 30
   ggaguauguu ucuguggag 19
<210> 31
   <211> 19
   <212> ARN
   <213> Séquence artificielle
<220>
   <223> siRNA
<400> 31
   gaguauguuu cuguggaga 19